(19) Europäisches Patentamt / European Patent Office / Office européen des brevets

(11) **EP 3 844 503 B1**

(12) **EUROPEAN PATENT SPECIFICATION**

(45) Date of publication and mention of the grant of the patent:
**29.05.2024 Bulletin 2024/22**

(21) Application number: **19762354.9**

(22) Date of filing: **30.08.2019**

(51) International Patent Classification (IPC):
**G01N 33/574** (2006.01)    **C12Q 1/48** (2006.01)

(52) Cooperative Patent Classification (CPC):
**G01N 33/57407; C12Q 1/485; G01N 33/48; G01N 33/54313; G01N 33/552;** G01N 2800/52

(86) International application number:
**PCT/EP2019/073180**

(87) International publication number:
**WO 2020/043868 (05.03.2020 Gazette 2020/10)**

(54) **THYMIDINE KINASE (TK-1) IN PROGNOSTIC INDICES FOR DLBCL**

THYMIDINKINASE (TK-1) IN PROGNOSTISCHEN INDIZES FÜR DLBCL

THYMIDINE KINASE (TK-1) DANS LA DÉTERMINATION D'INDICES DE PRONOSTIC POUR LE DLBCL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.08.2018 US 201816118572**

(43) Date of publication of application:
**07.07.2021 Bulletin 2021/27**

(73) Proprietors:
• **F. Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC MK MT NL NO PL PT RO RS SE SI SK SM TR**
• **Roche Diagnostics GmbH**
  **68305 Mannheim (DE)**
  Designated Contracting States:
  **DE**

(72) Inventors:
• **KLAMMER, Martin**
  **82377 Penzberg (DE)**
• **MORGENSTERN, David**
  **Indianapolis, Indiana 46250-0457 (US)**
• **PINCHUK, Boris**
  **82377 Penzberg (DE)**
• **ROLNY, Vinzent**
  **82377 Penzberg (DE)**
• **RUTZ, Sandra**
  **82377 Penzberg (DE)**
• **SONNER, Franziska**
  **82377 Penzberg (DE)**
• **ZIMMERMANN, Christina**
  **82377 Penzberg (DE)**

(74) Representative: **Simmons & Simmons LLP (Munich) et al**
  **Lehel Carré**
  **Thierschplatz 6**
  **80538 Munich (DE)**

(56) References cited:
• **KAZUHITO SUZUKI ET AL: "Prognostic value of high thymidine kinase activity in patients with previously untreated diffuse large B-cell lymphoma treated by rituximab, cyclophosphamide, doxorubicin, vincristine and prednisolone", LEUKEMIA AND LYMPHOMA.,** vol. 54, no. 11, 30 November 2013 (2013-11-30), **pages 2412-2417, XP055639478, US ISSN: 1042-8194, DOI: 10.3109/10428194.2013.779690**
• **SANDEEP K RAJAN: "Serum Thymidine Kinase (TK) Distinguishes Grade of Non Hodgkins Lymphoma (NHL) and Predicts Complete Response Rate (CRR) and Progression Free Survival (PFS). | Blood | American Society of Hematology", BLOOD,** vol. 104, no. 11, 16 November 2004 (2004-11-16), **page 4558, XP055640109,**

- **SAMER SUKI ET AL: "Risk Classification for Large Cell Lymphoma using Lactate Dehydrogenase, Beta-2 Microglobulin, and Thymidine Kinase", LEUKEMIA AND LYMPHOMA., vol. 18, no. 1-2, 1 January 1995 (1995-01-01), pages 87-92, XP55639937, US ISSN: 1042-8194, DOI: 10.3109/10428199509064927**

Remarks:

    The complete document including Reference Table(s) and the Sequence Listing(s) can be downloaded from the EPO website

**Description**

The present invention relates to the finding that thymidine kinase 1 (TK-1) represents a valuable biomarker in a method for determining a Prognostic Index (PI) for risk stratification of a patient with aggressive B-cell lymphoma, especially diffuse large B-cell lymphoma (DLBCL), to the use of TK-1 in such PI and to a PI comprising the marker TK-1.

The stratification of DLBCL patients into different risk groups is usually performed based on a prognostic index scoring. At present there are several Prognostic Indices (PIs) that are used for such patient stratification (Wight et al. Blood Reviews, 2018). NCCN guidelines include the following: IPI, age-adjusted IPI, stage-modified IPI and NCCN-IPI. ESMO guidelines recommend IPI and age-adjusted IPI.

The standard International Prognostic Index (IPI) [Ziepert M. et al., Journal of Clinical Oncology: 28/14 (2010) 2373] is widely used in the clinic to predict the outcome of DLBCL patients treated with R-CHOP (Rituximab plus CHOP chemotherapy). Also quite popular is the National Comprehensive Cancer Network International Prognostic Index (NCCN-IPI) [Zhou et al., Blood, 123, 6: (2014) 837].

While there are some differences regarding the parameters used for determining the different PIs used in the field of DLBCL, there is consensus that Ann Arbor stage, extranodal disease status and - as the only biomarker - lactate dehydrogenase (LDH) represent key elements of each of the PIs mentioned above.

As with all prognostic indices or scores improvements are highly desired, because the more accurate and reliable the index/score, the better the stratification of patients.

It was therefore the task of the inventors to the present disclosure to investigate whether there is a biomarker that may be used to improve the stratification of DLBCL patients, e.g. by determining a prognostic index or score.

This task is addressed in the present disclosure by providing the description, the examples and the embodiments as defined in the claims.

It has been found that the parameters extranodal disease status; Ann Arbor stage; and the level of thymidine kinase 1 are key to the determination of any prognostic index for patients with DLBCL.

Surprisingly it has also been found that the biomarker thymidine kinase 1 can not only replace the marker LDH in the PIs investigated but in combination with the other parameters used in a PI can lead to an improvement of such PI, i.e. to an improved patient stratification.

Kazuhito et al., Leukemia and Lymphoma 54(11), 2412-2417 (2013) describes the evaluation of 4 biological parameters as prognostic factors for overall survival (OS) among patients with DLBCL including thymidine kinase (TK) activity, wherein high TK activity was a strong predictor for short OS and poor response among patients.

**Summary of the Invention**

In one embodiment the present disclosure relates to a method of determining a prognostic index (PI) for a diffuse large B-cell lymphoma (DLBCL) patient according to claim 1, the method comprising a) determining at least the following parameters: i) extranodal disease status; ii) Ann Arbor stage; and iii) the level of thymidine kinase 1, wherein absence of extranodal disease values 0 points and presence of extranodal disease values 1 point, wherein an Ann Arbor stage of I or II values 0 points and an Ann Arbor stage of III or IV values 1 point, and wherein a level of the thymidine kinase up to and including the cut-off values 0 points and wherein a level of the thymidine kinase above cut-off values at least 1 point, and b) summing up the values for i), ii) and iii) in the determination of the PI.

**Detailed description**

In one embodiment the present disclosure relates to a method of determining a prognostic index (PI; also called risk score or PI-score) for a diffuse large B-cell lymphoma (DLBCL) patient according to claim 1, the method comprising

a) determining at least the following parameters:

i) extranodal disease status; ii) Ann Arbor stage; and iii) the level of thymidine kinase 1,
wherein absence of extranodal disease values 0 points and presence of extranodal disease values 1 point,
wherein an Ann Arbor stage of I or II values 0 points and an Ann Arbor stage of III or IV values 1 point, and
wherein a level of the thymidine kinase up to and including the cut-off values 0 points and wherein a level of the thymidine kinase above cut-off values at least 1 point, and

b) summing up the values for i), ii) and iii) in the determination of the PI.

Human thymidine kinase 1, (abbreviations: TK-1, hTK-1, hTK1 or TK1), (ATP: thymidine 5'- phosphotransferase, EC 2.7.1.21) is an enzyme involved in DNA precursor synthesis. Human thymidine kinase 1 consists of 234 amino acids as shown in SEQ ID NO:1. In the human organism TK-1 is present in various forms like dimers, tetramers and polymers of high molecular weight. These forms seem to depend on the presence of certain molecules, e.g. presence or absence of adenosine triphosphate (ATP); the concentration of the hTK-1 protein itself, the type of the protein, i.e. native or recombinant TK 1; and on the site/location of the protein, i.e. in serum or cytoplasm.

The level of thymidine kinase 1 in a sample of interest can be determined by any appropriate means. This determination is made in vitro. In one embodiment the level of thymidine kinase 1 is determined in vitro from a sample of interest.

In one embodiment the level of TK-1 is the enzymatic activity level. The serum TK-1 enzymatic activity can e.g. be measured using a radioactive substrate 1251-dUrd (the PROLIFIGEN® TK-REA, DiaSorin Inc.) or a non-radiometric substrate TK-1 activity assay (TK LIAISON® assay, DiaSorin Inc.). In one embodiment the level of thymidine kinase 1 is the protein level of thymidine kinase 1.

The term "cut-off" relates to a decision point, usually a value and/or concentration. Values below or at cut-off are grouped into a first group and values above cut-off are grouped into a second group. Sometimes more than one cut-off point is used. In this case values below or at the first cut-off are grouped into a first group, values above the first cut-off and up to and including the second cut-off are grouped into a second group, values above the second cut-off and up to and including etc.

It was quite a surprising finding that the level of thymidine kinase 1 in patients with DLBCL is way above the level of TK-1 measured in samples from healthy individuals. In one embodiment the cut-off is determined in base line samples of patients with newly diagnosed DLBCL and is in the range of the 25%-percentile to the 35%-percentile of newly diagnosed patients with DLBCL.

In one embodiment the cut-off value is determined based on samples taken from healthy controls. In one embodiment the thymidine kinase cut-off level is 4 times the mean value of the TK-1 level as determined in healthy individuals.

It has been found that it is possible to use a single cut-off point for TK-1 and to build two groups of patients, i.e. those with a thymidine kinase 1 level up to and including the cut-off values and those with a TK-1 level above the cut-off.

In one embodiment the present disclosure relates to a method for determining a PI as stipulated above, wherein the thymidine kinase 1 cut-off level is set to 4 times the mean value of the TK-1 level as determined in healthy individuals, wherein a thymidine kinase 1 level up to and including the cut-off values 0 points and wherein any TK-1 level above cut-off values 1 point.

It has further been found that it may be advantageous to build two groups of patients based on their level of TK-1, i.e. those with a low thymidine kinase 1 level, i.e. a TK-1 level of up to and including the cut-off value, an intermediate group, i.e. those with a TK-1 level above the cut-off and up to and including the value corresponding to 20 times the mean value as determined in healthy individuals and those with a high thymidine kinase level, i.e. a level of TK-1 of more than 20 times the mean value as determined in healthy individuals.

In one embodiment the present disclosure relates to a method for determining a PI as stipulated above, wherein the thymidine kinase 1 cut-off level is set to 4 times the mean value of the TK-1 level as determined in healthy individuals, wherein a low thymidine kinase 1 level, i.e. a level of TK-1 up to and including the cut-off values 0 points, an intermediate thymidine kinase level, i.e. a level of TK-1 above the cutoff and up to and including the value corresponding to 20 times the mean value as determined in healthy individuals values 1 point, and a high thymidine kinase level, i.e. a level of TK-1 of more than 20 times the mean value as determined in healthy individuals values 2 points.

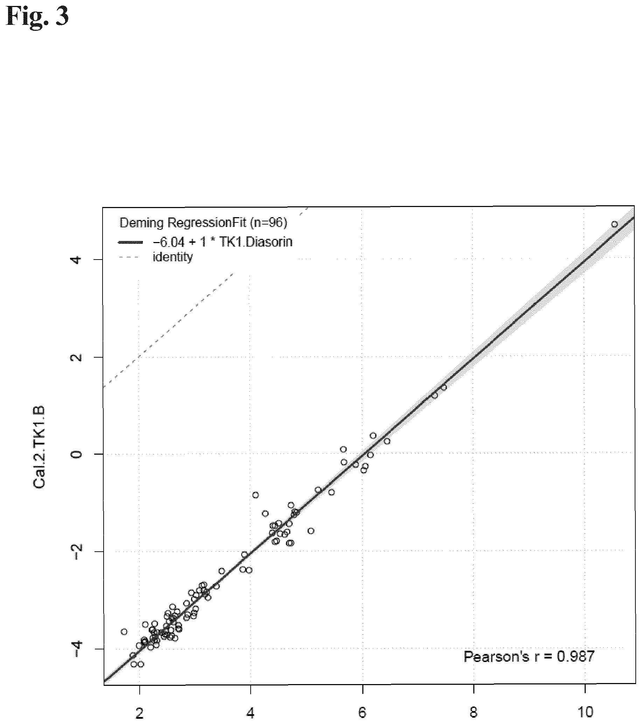

It has been found that the TK-1 level in terms of protein concentration, determined with a not yet fully standardized immunoassay, and the TK-1 enzymatic activity level, determined with the commercially available enzyme activity assay from DiaSorin, show a very good correlation (cf. Figure 3). It is therefore also possible to give a cut-off-value in terms of enzymatic activity (U/l).

In one embodiment the present disclosure relates to a method for determining a PI, wherein the cut-off on the protein level for TK-1 is equivalent to and includes 18 U/l in TK-1 enzymatic activity.

In one embodiment the present disclosure relates to a method for determining a PI, wherein the cut-off on the protein level is equivalent to and includes 18 U/l and wherein a level of TK-1 above 18 U/l and up to and including 88 U/l values 1 point and a level of TK-1 above 88 U/l values 2 points.

It has been found that for any patient, especially for those recently diagnosed with DLBCL, TK-1 should be measured and compared to a pre-defined cutoff. If the measured value for the newly diagnosed patient is above the predefined cutoff (binarized approach), the patient would be considered to have a higher risk score and be more likely to experience an unfavorable disease outcome.

It is also possible to define more than two risk groups based on an increasing set of cutoffs, e.g. in a trichotomized approach using 0 (risk) points in the determination of a PI for the patients with a low level of TK-1, 1 (risk) point in the determination of a PI for the patients with an intermediate level of TK-1 and 2 (risk) points in the determination of a PI for the patients with a high level of TK-1. A patient would then be assigned to one of the risk groups based on the value of his TK-1 measurement; the higher the risk score the more unfavorable disease outcome.

Alternatively, it would be also possible to transform the TK-1 measurement values directly into a continuous risk score based on a pre-defined suitable transformation function (e.g. log2-transformation). In one embodiment in a method according to the present disclosure the PI is determined based on TK-1 measurement values that have been log2-transformed. In one embodiment in a method according to the present disclosure the PI is determined based on a continuous risk score for TK-1 measurement values. Any of these stratified or transformed TK-1 values can be added to any of the existing prognostic indices (either with or without replacement of LDH).

The method for determining a prognostic index, as disclosed herein, i.e. comprising the parameters extranodal disease status; Ann Arbor stage; and the level of thymidine kinase 1, further comprises the parameter age, wherein if the age is below cut-off, the value is 0 points and if the age is above cut-off, the value is 1 point in the determination of the PI.

The method for determining a prognostic index, as disclosed herein, i.e. comprising the parameters extranodal disease status; Ann Arbor stage; and the level of thymidine kinase 1, further comprises the parameter ECOG performance status, wherein, in case the ECOG performance status 1 or below, the value is 0 points and wherein, in case the ECOG performance status is 2 or above, the value is 1 point in the determination of the PI.

At least four different but related prognostic indices for DLBCL are currently available and used in clinical practice, International Prognostic Index (IPI), the "Age-adjusted IPI", the Revised Standard International Prognostic Index (R-IPI), and the National Comprehensive Cancer Network International Prognostic Index (NCCN-IPI).

A "Prognostic Index" (PI) (also called risk score or PI-score) provides a numeric value. The numeric value of such PI is e.g. generated by summing up the values determined for each of the individual parameters (components) of such PI. In one embodiment the method of determining a PI according to the present disclosure is implemented in a computer-readable program. In one embodiment the method of determining a PI according to the present disclosure is implemented into an App. The prognostic index or PI-score can e.g. be used to split/stratify patients into groups. Such groups for example can be groups with different risk of disease progression. Usually about three or four groups are built; a low risk group, an intermediate risk group, and a high risk group, or a low risk group, an intermediate low risk group, an intermediate high risk group and a high risk group, respectively.

The "original" international prognostic index (IPI) in the field of DLBCL has been described in 1993 by Shipp MA and Harrington DP, "A predictive model for aggressive non-Hodgkin's lymphoma"; The international non-Hodgkin's Lymphoma prognostic factors project; N. Engl. J. Med. 1993;329(14):987-94. In this paper also the age-adjusted IPI, grouping patients into those above 60 and those up to and including 60, has been described. The disclosure of this reference is explicitly herewith included by reference.

The "original" international prognostic index (IPI) comprises the parameters age (above 60 = 1 point), ECOG performance status (2 or more = 1 point), Ann Arbor stage (III or IV = 1 point), LDH (above ULN (upper limit of normal) = 1 point) and extranodal sites (more than 1 = 1 point). The risk groups resulting from the IPI-scores are as follows: low risk group (0 to 1 point); low intermediate risk (2 points); high intermediate risk (3 points); and high risk (4 or 5 points).

The least complex version of the age-adjusted IPI is applied to patients of above 60. It comprises the parameters Ann Arbor stage (III or IV = 1 point); LDH (above ULN = 1 point) and extranodal sites (more than 1 = 1 point). Four risk groups are determined, i.e., low risk group (0 points); low intermediate risk (1 point); high intermediate risk (2 points); and high risk (3 points).

The age-adjusted IPI as applied to patients of up to and including 60 also comprises the parameters Ann Arbor stage (III or IV = 1 point); LDH (above ULN = 1 point) and extranodal sites (more than 1 = 1 point), but uses a slightly different scoring. The four risk groups here are, low risk group (0 to 1 point); low intermediate risk (1 to 2 points); high intermediate risk (2 to 3 points); and high risk (4 points).

As found by the inventors to the present invention, TK-1 can be used to substitute for LDH both in IPI as well as in the age-adjusted IPI.

The (Revised) International Prognostic Index (R-IPI) [Sehn, et al., 2007] is widely used in the clinic to predict the outcome of DLBCL patients treated with R-CHOP (Rituximab plus CHOP chemotherapy). It comprises five components, which are summed-up for the final risk score (Table 1).

*Table 1: Components and thresholds of the R-IPI prognostic index.*

| R-IPI components | Score |
|---|---|
| Age > 60 | 1 |
| ECOG performance status >= 2 | 1 |
| Lactate dehydrogenase (LDH) elevated | 1 |
| Ann Arbor Stage III-IV | 1 |
| Number of extranodal sites > 1 | 1 |

In one embodiment the method disclosed in the present invention for determining a PI, i.e. comprising the parameters extranodal disease status; Ann Arbor stage; and the level of thymidine kinase 1, further includes the parameter age and the parameter Ann Arbor stage, wherein if the age is below and including 60, the value is 0 points, wherein if the age is above 60, the value is 1 point in the determination of the PI, wherein, in case the Ann Arbor stage is II or below the value is 0 points, and wherein, in case the Ann Arbor stage is III or IV, the value is 1 point in the determination of the PI. In one embodiment of this method extranodal disease is disease in more than 1 extranodal site (including bone marrow) and, if met, scores 1 point in the determination of the PI. In one embodiment the present invention relates to an improved R-IPI, comprising the above parameters (components) from Table 1, wherein the level of TK-1 is used instead of the level of LDH. In one embodiment in the improved R-IPI the thymidine kinase 1 cut-off level is set to 4 times the mean value of the TK-1 level as determined in healthy individuals, wherein a thymidine kinase 1 level up to and including the cut-off values 0 points and wherein any TK-1 level above cut-off values 1 point. In one embodiment in the improved R-IPI the thymidine kinase cut-off level is set to 4 times the mean value of the TK-1 level as determined in healthy individuals and a low thymidine kinase level, i.e. a level of TK-1 up to and including the cut-off values 0 points, an intermediate thymidine kinase level, i.e. a level of TK-1 above the cut-off and up to and including the value corresponding to 20 times the mean value as determined in healthy individuals values 1 point, and a high thymidine kinase level, i.e. a level of TK-1 of more than 20 times the mean value as determined in healthy individuals values 2 points.

The "TK-1 IPI" (IPI using TK-1 instead of LDH) risk groups stay as published if a single cut-off point for the level of TK-1 as described above is used, i.e., low risk group (0 to 1 point); low intermediate risk (2 points); high intermediate risk (3 points); and high risk (4 or 5 points). The prognostic capability of "TK-1 IPI" (IPI using TK-1 instead of LDH) can be further improved by trichotomizing the values determined for TK-1 level. The corresponding risk groups are: low risk group (0 points); low intermediate risk (1 point); high intermediate risk (2 to 3 points); and high risk (4 to 6 points).

The "TK-1 R-IPI" (R-IPI using TK-1 instead of LDH) risk groups stay as published if a single cut-off point for the level of TK-1 as described above is used, i.e., low risk group ("very good"; 0 points); intermediate risk ("good"; 1 to 2 points); high risk ("poor" 3 to 5 points). The prognostic capability of "TK-1 R-IPI" (R-IPI using TK-1 instead of LDH) can be further improved by trichotomizing the values determined for TK-1 level. The corresponding risk groups are: low risk group ("very good"; 0 points); intermediate risk ("good"; 1 to 3 points); high risk ("poor" 4 to 6 points).

The National Comprehensive Cancer Network International Prognostic Index (NCCN-IPI) is a derivative of the R-IPI and contains the following risk components (Table 2).

*Table 2: Components and thresholds of the NCCN-IPI prognostic index. ULN = upper limit of normal*

| NCCN-IPI components | Score |
|---|---|
| **Age** | |
| >40 to <=60 | 1 |
| >60 to <=75 | 2 |
| >75 | 3 |
| **LDH normalized [ULN]** | |
| >1 to <=3 | 1 |
| >3 | 2 |
| Ann Arbor Stage III-IV | 1 |
| Extranodal disease (disease in bone marrow, CNS, liver, GI tract, lung) | 1 |
| ECOG performance status >= 2 | 1 |

In one embodiment the method disclosed in the present invention for determining a PI, i.e. comprising the parameters extranodal disease status; Ann Arbor stage; and the level of thymidine kinase 1, further includes the parameter age and the parameter ECOG performance status, wherein if the age is below and including 40, the value is 0 points, wherein if the age is above 40 and up to and including 60, the value is 1 point, wherein if the age is above 60 and up to and including 75, the value is 2 points in the determination of the PI and wherein if the age is above 75 the value is 3 points in the determination of the PI, and wherein, in case the ECOG performance status is 1 or below the value is 0 points, and wherein, in case the ECOG performance status is 2 or above, the value is 1 point in the determination of the PI. In one embodiment of this method extranodal disease is disease in 1 or more site selected from bone marrow, CNS, liver, GI tract and lung and scores 1 point, if this criterion is met. In one embodiment the present invention relates to an improved NCCN-IPI, comprising the above parameters (components) from Table 2, wherein the level of TK-1 is used instead of the level of LDH. In one embodiment in the improved NCCN-IPI the thymidine kinase cut-off level is set to 4 times the mean value of the TK-1 level as determined in healthy individuals and a low thymidine kinase level, i.e. a level of TK-1 up to and including the cut-off values 0 points, an intermediate thymidine kinase level, i.e. a level of TK-1 above the cut-off and up to and including the value corresponding to 20 times the mean value as determined in healthy individuals values 1 point, and a high thymidine kinase level, i.e. a level of TK-1 of more than 20 times the mean value as determined in healthy individuals values 2 points.

The "TK-1 NCCN-IPI" (NCCN-IPI using TK-1 instead of LDH) risk groups stay as published, i.e., low risk group (0 to 1 point); low intermediate risk (2 to 3 points); high intermediate risk (4 to 5 points); and high risk (6 points and above).

As obvious from Tables 1 and 2 as well as from the description of the "older" IPIs above there is consensus that Ann Arbor stage and extranodal disease and - as the only biomarker - lactate dehydrogenase (LDH) represent key elements of each of the PIs for DLBCL mentioned above.

As can be seen for IPI, Age-adjusted IPI, R-IPI, and NCCN-IPI, the lactate dehydrogenase (LDH) value usually is determined by use of the upper limit of normal (ULN). An LDH level below and including the ULN values 0 points and an LDH-level above ULN values at least one point.

The Ann Arbor staging is performed as described in https://www.ncbi.nlm.nih.gov/pubmed/5121694 and https://www.ncbi.nlm.nih.gov/pubmed/2809679, the disclosure of which is hereby included in its entirety by reference.

The ECOG (Eastern Cooperative Oncology Group) performance status (= "ECOG status") is determined as defined and described by Oken MM, Creech RH, Tormey DC, et. al., Eastern Cooperative Oncology Group, Robert Comis M.D., Group Chair. Toxicity and response criteria of the Eastern Cooperative Oncology Group. Am J Clin Oncol. 1982 Dec;5(6):649-55.

All DLBCL PIs mentioned above use the same cut-off for ECOG-status (ECOG performance status), i.e. an ECOG status of 2 or above values 1 point in the determination of each of these indices, while an ECOD status of 0 or 1 values 0 points. The same applies to the ECOG status as part of method or prognostic index disclosed herein.

There is broad consent that "extranodal disease" represents yet one further central element of any of the above mentioned PIs. There are minor differences regarding the definition of extranodal disease in between the various IPIs described in more detail above. As obvious from the Examples given, the value for the level of TK-1 can be used and combined with any of the PIs described above. Therefor in one embodiment the present invention relates to a method of determining a PI or to a PI as such, wherein the definition for extranodal disease is selected from disease in more than 1 extranodal site (including bone marrow) or from disease (1 or more) in bone marrow, CNS, liver, GI tract, lung.

The term "sample" or "sample of interest" or "test sample" are used interchangeably herein. The sample is an in vitro sample, it will be analyzed in vitro and not transferred back into the body. In one embodiment the sample of interest is taken from a patient newly diagnosed as suffering from DLBCL and before treatment is initiated. Examples of samples include but are not limited to fluid samples such as blood, serum, plasma, synovial fluid, urine, saliva, and lymphatic fluid, or solid samples such as tissue extracts, cartilage, bone, synovium, and connective tissue. In one embodiment the sample is selected from blood, serum, plasma, synovial fluid and urine. In one embodiment the sample is selected from blood, serum and plasma. In one embodiment the sample is serum or plasma.

The term "reference sample" as used herein, refers to a sample which is analyzed in a substantially identical manner as the sample of interest and whose information is compared to that of the sample of interest. A reference sample thereby provides a standard allowing for the evaluation of the information obtained from the sample of interest. A reference sample may be derived from a healthy or normal tissue, organ or individual, thereby providing a standard of a healthy status of a tissue, organ or individual. In one embodiment the reference samples for determining the mean value of TK-1 are derived from healthy individuals.

As indicated further above, the level of thymidine kinase used for determining a prognostic index in one embodiment is the protein level. Various ways to determine a protein level are available and within the skills of a person familiar in this field.

In one embodiment the protein level of thymidine kinase is determined in an immunoassay. Any type of immunoassay can be used to measure the TK-1 protein level in a sample. Examples of immunoassays which can be utilized to measure the TK-1 level for later use in the determination of a PI are the enzyme linked immunosorbent assay (ELISA), enzyme immunoassay (EIA), radioimmunoassay (RIA), or immunoassays based on detection of luminescence, chemiluminescence, electrochemiluminescence, or fluorescence.

One commercially available immunoassay is Arocell's hTK-1 assay which is based on a microtiter plate format.

In one embodiment the TK-1 level used to determine a prognostic index is measured in a sandwich assay format.

In a typical sandwich-type assay, a first antibody bound to the solid phase or capable of binding thereto and a detectably-labeled second antibody each bind to the analyte at different and non-overlapping epitopes. The first analyte-specific binding agent (e.g. an antibody) is either covalently or passively bound to a solid surface. The solid surface is typically glass or a polymer, the most commonly used polymers being cellulose, polyacrylamide, nylon, polystyrene, polyvinyl chloride, or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes or overnight if more convenient) and under suitable conditions (e.g., from room temperature to 40°C such as between 25° C and 37° C inclusive) to allow for binding between the first or capture antibody and the corresponding antigen. Following the incubation period, the solid phase, comprising the first or capture antibody and bound thereto the antigen can be washed, and incubated with a secondary or labeled antibody binding to another epitope on the antigen. The second antibody is linked to a reporter molecule which is used to indicate the binding of the second antibody to the complex of first antibody and the antigen of interest.

An extremely versatile alternative sandwich assay format includes the use of a solid phase coated with the first partner of a binding pair, e.g. paramagnetic streptavidin-coated microparticles. Such microparticles are mixed and incubated with an analyte-specific binding agent bound to the second partner of the binding pair (e.g. a biotinylated antibody), a sample suspected of comprising or comprising the analyte, wherein said second partner of the binding pair is bound to said analyte-specific binding agent, and a second analyte-specific binding agent which is detectably labeled, e.g. with an electrochemiluminescent label as used herein. As obvious to the skilled person these components are incubated under appropriate conditions and for a period of time sufficient for binding the labeled antibody via the analyte, the analyte-specific binding agent (bound to) the second partner of the binding pair and the first partner of the binding pair to the solid phase microparticles. As appropriate such assay may include one or more washing step(s).

As obvious to the skilled artisan the sample can be contacted with the first and the second antibody in any desired order, i.e. first antibody first, the second antibody; second antibody first than first antibody, or simultaneously, for a time and under conditions sufficient to form a first anti-hTK-1 antibody/hTK-1/second anti-hTK-1 antibody complex.

As the skilled artisan will readily appreciate it is nothing but routine experimentation to establish the time and conditions that are appropriate or that are sufficient for the formation of a complex either between the specific anti hTK-1 antibody and the hTK-1 antigen/analyte (=anti-hTK-1 antibody/hTK-1 complex) or the formation of the secondary or sandwich complex comprising the first antibody to hTK-1, the hTK-1 (the analyte) and the second anti-hTK-1 antibody complex (=first anti-hTK-1 antibody/hTK-1/second anti-hTK-1 antibody complex).

The detection of the anti-hTK-1 antibody/hTK-1 complex can be performed by any appropriate means. The person skilled in the art is absolutely familiar with such means/methods.

In an immunoassay method for measuring the level of hTK-1 usually at least one antibody to hTK-1 comprises a detectable label.

The term detectably labeled encompasses labels that can be directly or indirectly detected.

Directly detectable labels either provide a detectable signal or they interact with a second label to modify the detectable signal provided by the first or second label, e.g. to give FRET (fluorescence resonance energy transfer). Labels such as fluorescent dyes and luminescent (including chemiluminescent and electrochemiluminescent) dyes (Briggs et al "Synthesis of Functionalised Fluorescent Dyes and Their Coupling to Amines and Amino Acids," J. Chem. Soc., Perkin-Trans. 1 (1997) 1051-1058) provide a detectable signal and are generally applicable for labeling. In one embodiment detectably labeled refers to a label providing or inducible to provide a detectable signal, i.e. to a fluorescent label, to a luminescent label (e.g. a chemiluminescent label or an electrochemiluminescent label), a radioactive label or a metal-chelate based label, respectively.

Numerous labels (also referred to as dyes) are available which can be generally grouped into the following categories, all of them together and each of them representing embodiments according the present disclosure:

(a) Fluorescent dyes

Fluorescent dyes are e.g. described by Briggs et al "Synthesis of Functionalized Fluorescent Dyes and Their Coupling to Amines and Amino Acids," J. Chem. Soc., Perkin-Trans. 1 (1997) 1051-1058).

Fluorescent labels or fluorophores include rare earth chelates (europium chelates), fluorescein type labels including FITC, 5-carboxyfluorescein, 6-carboxy fluorescein; rhodamine type labels including TAMRA; dansyl; Lissamine; cyanines; phycoerythrins; Texas Red; and analogs thereof. The fluorescent labels can be conjugated to an aldehyde group comprised in target molecule using the techniques disclosed herein. Fluorescent dyes and fluorescent label reagents include those which are commercially available from Invitrogen/Molecular Probes (Eugene, Oregon, USA) and Pierce Biotechnology, Inc. (Rockford, Ill.).

(b) Luminescent dyes

Luminescent dyes or labels can be further subcategorized into chemiluminescent and electrochemiluminescent dyes.

The different classes of chemiluminogenic labels include luminol, acridinium compounds, coelenterazine and analogues, dioxetanes, systems based on peroxyoxalic acid and their derivatives. For immunodiagnostic procedures predominantly acridinium based labels are used (a detailed overview is given in Dodeigne C. et al., Talanta 51 (2000) 415-439).

The labels of major relevance used as electrochemiluminescent labels are the Ruthenium- and the Iridium-based electrochemiluminescent complexes, respectively. Electrochemiluminescence (ECL) proved to be very useful in analytical applications as a highly sensitive and selective method. It combines analytical advantages of chemiluminescent analysis (absence of background optical signal) with ease of reaction control by applying electrode potential. In general Ruthenium complexes, especially [Ru (Bpy)3]2+ (which releases a photon at ~620 nm) regenerating with TPA (Tripropylamine) in liquid phase or liquid-solid interface are used as ECL-labels.

Electrochemiluminescent (ECL) assays provide a sensitive and precise measurement of the presence and concentration of an analyte of interest. Such techniques use labels or other reactants that can be induced to luminesce when electrochemically oxidized or reduced in an appropriate chemical environment. Such electrochemiluminescence is triggered by a voltage imposed on a working electrode at a particular time and in a particular manner. The light produced by the label is measured and indicates the presence or quantity of the analyte. For a fuller description of such ECL techniques, reference is made to US Patent No. 5,221,605, US Patent No. 5,591,581, US Patent No. 5,597,910, PCT published application WO90/05296, PCT published application WO92/14139, PCT published application WO90/05301, PCT published application WO96/24690, PCT published application US95/03190, PCT application US97/16942, PCT published application US96/06763, PCT published application WO95/08644, PCT published application WO96/06946, PCT published application WO96/33411, PCT published application WO87/06706, PCT published application WO96/39534, PCT published application WO96/41175, PCT published application WO96/40978, PCT/US97/03653 and US patent application 08/437,348 (U.S. Patent No. 5,679,519). Reference is also made to a 1994 review of the analytical applications of ECL by Knight, et al. (Analyst, 1994, 119: 879-890) and the references cited therein. In one embodiment the method according to the present description is practiced using an electrochemiluminescent label.

Recently also Iridium-based ECL-labels have been described (WO2012107419(A1)).

In one embodiment the directly detectable label is a chemiluminescent or an electrochemiluminescent label. The light produced by the label is measured and directly or indirectly indicates the presence or quantity of the analyte.

(c) Radioactive labels make use of radioisotopes (radionuclides), such as 3H, 11C, 14C, 18F, 32P, 35S, 64Cu, 68Gn, 86Y, 89Zr, 99TC, 111In, 123I, 124I, 125I, 131I, 133Xe, 177Lu, 211At, or 131Bi.

(d) Metal-chelate complexes suitable as labels for imaging and therapeutic purposes are well-known in the art (US 2010/0111856; US 5,342,606; US 5,428,155; US 5,316,757; US 5,480,990; US 5,462,725; US 5,428,139; US 5,385,893; US 5,739,294; US 5,750,660; US 5,834,456; Hnatowich et al, J. Immunol. Methods 65 (1983) 147-157; Meares et al, Anal. Biochem. 142 (1984) 68-78; Mirzadeh et al, Bioconjugate Chem. 1 (1990) 59-65; Meares et al, J. Cancer (1990), Suppl. 10:21-26; Izard et al, Bioconjugate Chem. 3 (1992) 346-350; Nikula et al, Nucl. Med. Biol. 22 (1995) 387-90; Camera et al, Nucl. Med. Biol. 20 (1993) 955-62; Kukis et al, J. Nucl. Med. 39 (1998) 2105-2110; Verel et al., J. Nucl. Med. 44 (2003) 1663-1670; Camera et al, J. Nucl. Med. 21 (1994) 640-646; Ruegg et al, Cancer Res. 50 (1990) 4221-4226; Verel et al, J. Nucl. Med. 44 (2003) 1663-1670; Lee et al, Cancer Res. 61 (2001) 4474-4482; Mitchell, et al, J. Nucl. Med. 44 (2003) 1105-1112; Kobayashi et al Bioconjugate Chem. 10 (1999) 103-111; Miederer et al, J. Nucl. Med. 45 (2004) 129-137; DeNardo et al, Clinical Cancer Research 4 (1998) 2483-90; Blend et al, Cancer Biotherapy & Radiopharmaceuticals 18 (2003) 355-363; Nikula et al J. Nucl. Med. 40 (1999) 166-76; Kobayashi et al, J. Nucl. Med. 39 (1998) 829-36; Mardirossian et al, Nucl. Med. Biol. 20 (1993) 65-74; Roselli et al, Cancer Biotherapy & Radiopharmaceuticals, 14 (1999) 209-20).

The immunoassay used in the Examples section employed some newly developed antibodies which are not publically available yet and therefore described in some detail below. The antibodies used both specifically bind to human thymidine kinase 1 (hTK-1) of SEQ ID NO:1.

The term "specifically binds" (also referred to herein as "specifically interacts"), in accordance with the present invention, means that the antibody specifically binds only hTK-1, but does not or essentially does not cross-react with a different protein, in particular a different protein of similar structure such as e.g. thymidine kinase 2 (SEQ ID NO:5).

Corresponding methods for analyzing the specificity of an antibody are described e.g. in Harlow & Lane (1988) Antibodies: A Laboratory Manual, Cold Spring Harbor Laboratory Press, and in Harlow & Lane (1999) Using Antibodies: A Laboratory Manual, Cold Spring Harbor Laboratory Press. Non-limiting examples of suitable studies are e.g. binding studies, blocking and competition studies with structurally and/or functionally closely related molecules. These studies can be carried out by methods such as e.g. FACS analysis, flow cytometric titration analysis (FACS titration), surface plasmon resonance (SPR, e.g. with BIAcore®), isothermal titration calorimetry (ITC), fluorescence titration, or by radiolabeled ligand binding assays. Further methods include e.g. Western Blots, ELISA (including competition ELISA)-, RIA-, ECL-, and IRMA-tests.

In context of the present invention, the term "antibody" relates to full immunoglobulin molecules as well as to antigen binding fragments thereof, like, Fab, Fab', F(ab')₂, Fv. Furthermore, the term relates to modified and/or altered antibody molecules, as well as to recombinantly or synthetically generated/synthesized antibodies. The term "antibody" also comprises bifunctional antibodies, trifunctional antibodies, fully-human antibodies, chimeric antibodies, and antibody constructs, like single chain Fvs (scFv) or antibody-fusion proteins.

A "Fab fragment" as used herein is comprised of one light chain and the $C_H1$ and variable regions of one heavy chain. The heavy chain of a Fab molecule cannot form a disulfide bond with another heavy chain molecule. A "Fab' fragment" contains one light chain and a portion of one heavy chain that contains the $V_H$ domain and the $C_H1$ domain and also the region between the $C_H1$ and $C_H2$ domains, such that an interchain disulfide bond can be formed between the two heavy chains of two Fab' fragments to form a F(ab')$_2$ molecule. A "F(ab')$_2$ fragment" contains two light chains and two heavy chains containing a portion of the constant region between the $C_H1$ and $C_H2$ domains, such that an interchain disulfide bond is formed between the two heavy chains. A F(ab')$_2$ fragment thus is composed of two Fab' fragments that are held together by a disulfide bond between the two heavy chains.

Fab/c fragment contain both Fc and Fab determinants, wherein an "Fc" region contains two heavy chain fragments comprising the $C_H2$ and $C_H3$ domains of an antibody. The two heavy chain fragments are held together by two or more disulfide bonds and by hydrophobic interactions of the $C_H3$ domains.

The "Fv region" comprises the variable regions from both the heavy and light chains, but lacks the constant regions. "Single-chain Fvs" (also abbreviated as "scFv") are antibody fragments that have, in the context of the present invention, the $V_H$ and $V_L$ domains of an antibody, wherein these domains are present in a single polypeptide chain. Generally, the scFv polypeptide further comprises a polypeptide linker between the $V_H$ and $V_L$ domains which enables the scFv to form the desired structure for antigen binding. Techniques described for the production of single chain antibodies are described, e.g., in Pliickthun in The Pharmacology of Monoclonal Antibodies, Rosenburg and Moore eds. Springer-Verlag, N.Y. 113 (1994), 269-315.

The term "chimeric antibodies" refers to antibodies that comprise a variable region of a human or non-human species fused or chimerized to an antibody region (e.g., constant region) from another species, either human or non-human (e.g., mouse, horse, rabbit, dog, cow, chicken).

As mentioned above, the term "antibody" also encompasses antibody constructs, such as antibody-fusion proteins, wherein the antibody comprises (an) additional domain(s), e.g. for the isolation and/or preparation of recombinantly produced constructs, in addition to the domains defined herein by specific amino acid sequences.

An antibody can be produced such that it is a recombinant antibody, for example a recombinant rabbit antibody, or a hetero-hybrid antibody, yet comprising the CDRs as disclosed and defined in the present invention.

The term "recombinant antibody" includes all antibodies that are prepared, expressed, created or isolated by recombinant means. Recombinant antibodies are e.g. antibodies obtained by B-cell PCR, or antibodies isolated from an animal (e.g., a mouse) that is transgenic for human immunoglobulin genes, antibodies expressed using a recombinant expression vector transfected into a host cell, antibodies isolated from a recombinant, combinatorial human antibody library, or antibodies prepared, expressed, created or isolated by any other means that involves splicing of human immunoglobulin gene sequences to other DNA sequences. Recombinant rabbit antibodies as produced by B-cell PCR have variable and constant regions (if present) derived from rabbit germline immunoglobulin sequences. I.e. the direct result of B-cell PCR are the binding relevant fragments of an antibody and the skilled artisan has no problem whatsoever to e.g. construe a full length antibody, a chimeric antibody, or whatever "antibody" that will be desired/required.

One monoclonal antibody, MAB 6C6, has been found to be a very attractive tool for measuring TK-1 on the protein level via an immunoassay. MAB 6C6 is characterized by a heavy chain variable domain of SEQ ID NO:3 and a light chain variable domain of SEQ ID NO:4. As mentioned above, in one embodiment an antibody or antigen binding fragment thereof employed in an immunoassay for measurement of the TK-1 level, e.g. as shown in the Examples section, binds to the same epitope as an antibody comprising variable heavy chain (vHC) of SEQ ID NO:3 and a variable light chain (vLC) of SEQ ID NO:4.

Any monoclonal antibody or antigen binding fragment thereof binding to the same epitope as an antibody comprising variable heavy chain (vHC) of SEQ ID NO:3 and a variable light chain (vLC) of SEQ ID NO:4 can be employed in an immunoassay for measurement of TK-1. Such antibody as well as any antibody having the same binding properties, may e.g. be a variant of the specific antibody given and, e.g., may comprise amino acid substitutions, or in the alternative may have a different vHC or a different vLC or both.

The term "substitution", in accordance with the present invention, refers to the replacement of an amino acid with another amino acid. Thus, the total number of amino acids remains the same. The deletion of an amino acid at a certain position and the introduction of one (or more) amino acid(s) at a different position is explicitly not encompassed by the term "substitution". Substitutions, in accordance with the present invention, can be conservative amino acid substitutions or non-conservative amino acid substitutions. The term "conservative amino acid substitution" is well known in the art and refers to the replacement of an amino acid with a different amino acid having similar structural and/or chemical properties. Such similarities include e.g. a similarity in polarity, charge, solubility, hydrophobicity, hydrophilicity, and/or the amphipathic nature of the residues involved. The amino acid substitution is a conservative amino acid substitutions, in case one amino acid of one of the following groups is substituted by another amino acid of the same group: nonpolar (hydrophobic) amino acids include alanine, valine, leucine, isoleucine, proline, phenylalanine, tyrosine, tryptophan, and methionine; polar neutral amino acids include glycine, serine, threonine, cysteine, asparagine, and glutamine;

positively charged (basic) amino acids include arginine, lysine, and histidine; and negatively charged (acidic) amino acids include aspartic acid and glutamic acid.

The "binding affinity" of an antibody measures the strength of interaction between an epitope on the target antigen and the binding site of the antibody according to the following equation:

$$KD = kd/ka$$

wherein:

KD = dissociation equilibrium constant [M]
kd = dissociation rate constant [$s^{-1}$]
ka = association rate constant [$M^{-1}\ s^{-1}$]

Further relevant parameters for the binding affinity of an antibody are as follows:

$$t/2 = \text{dissociation complex half-life} = \ln2/kd/60\ [\min]$$

$$Rmax = \text{response maximum of analyte [RU]}$$

Mr:

$$\text{Molar Ratio} = \text{ratio of response maximum (Rmax) of analyte}$$

In one embodiment the monoclonal antibody to hTK-1 used for immunological detection of TK-1 level for determining a PI, binds to hTK-1 with a t/2-diss at 37°C of 10 minutes or longer.

Antibodies that bind to the same epitope on hTK-1 are antibodies that compete for binding to hTK-1 with an antibody having a heavy chain variable domain of SEQ ID NO:3 and a light chain variable domain of SEQ ID NO:4.

Such competing antibodies can be identified based on their ability to compete with monoclonal rabbit antibody MAB 6C6, i.e. an antibody comprising a heavy chain variable domain of SEQ ID NO:3 and a light chain variable domain of SEQ ID NO:4, in standard hTK-1 binding assays. For example, BIAcore analysis, ELISA assays or flow cytometry may be used to demonstrate competition with a monoclonal antibody or a binding fragment thereof having a heavy chain variable domain of SEQ ID NO:3 and a light chain variable domain of SEQ ID NO:4.

The ability of a test antibody to inhibit the binding of, monoclonal rabbit antibody MAB 6C6 to human TK-1 demonstrates that the test antibody can compete with monoclonal rabbit antibody MAB 6C6 for binding to and thus binds to the same epitope on hTK-1 as monoclonal rabbit antibody MAB 6C6.

As mentioned, several different competition assays may be used to identify an antibody that competes with rabbit monoclonal antibody MAB 6C6 for a binding fragment thereof having a heavy chain variable domain of SEQ ID NO:3 and a light chain variable domain of SEQ ID NO:4.

In an exemplary competition assay, immobilized hTK-1 is incubated in a solution comprising a first labeled antibody that binds to hTK-1 (e.g., an anti-hTK-1 monoclonal antibody or a binding fragment thereof having a heavy chain variable domain of SEQ ID NO:3 and a light chain variable domain of SEQ ID NO:4) and a second unlabeled antibody that is being tested for its ability to compete with the first antibody for binding to hTK-1. The second antibody may be present in a hybridoma supernatant. As a control, immobilized hTK-1 is incubated in a solution comprising the first labeled antibody but not the second unlabeled antibody. After incubation under conditions permissive for binding of the first antibody to hTK-1, excess unbound antibody is removed, and the amount of label associated with immobilized hTK-1 is measured. If the amount of label associated with immobilized hTK-1 is substantially reduced in the test sample relative to the control sample, then that indicates that the second antibody is competing with the first antibody for binding to hTK-1. See, e.g., Harlow et al. Antibodies: A Laboratory Manual. Ch.14 (Cold Spring Harbor Laboratory, Cold Spring Harbor, NY, 1988).

Binding properties of an antibody, e.g., of an anti-hTK-1 antibody, are best determined via real time biosensor-based molecular interaction measurements, like surface plasmon resonance spectroscopy, for which Biacore technology became a synonym. In the Biacore system antibodies can also be analyzed for competitive binding to the same epitope (i.e. for binding to an identical epitope or an overlapping epitope). Experimental details are given in Example 3. In one embodiment the competition experiment to characterize an antibody for binding to the conformation dependent epitope as identified herein is performed on the BIAcore instrument as described in the Examples section.

In the prior art, usually a serum or plasma sample is pretreated in order to generate the tetrameric and enzymatically highly active form of hTK-1. The pretreatment of a sample and the measurement of hTK-1 from such pretreated sample represents a very attractive method, since this way e.g. a very good correlation to hTK-1 enzymatic activity can be achieved.

The selection of an appropriate pretreatment reagent is fully within the capabilities of a person skilled in the art. Such pretreatment reagent will at least comprise a reducing agent in order to transform oligomeric hTK-1 into tetrameric hTK-1. As described e.g. by Sharif et al., (BMC Biochemistry (2012), 13:12) it is easy to assess via gel filtration experiments whether the reducing agent has been effective and hTK-1 is - after treatment - primarily present as a tetramer. Several different reducing reagents are at stake for the skilled artisan, e.g. dithiothreitol (DTT), dithioerythritol (DTE), or dithiobutylamin (DTBA). The concentration of the pretreatment reagent will in addition be chosen such way that either after an appropriate time of incubation or after a dilution step it will not negatively impact any antibody used to measure the hTK-1 protein. In case dithiobutylamin (DTBA) is used as a reducing agent an appropriate final concentration in the sample pretreatment step (the mixture of sample and pretreatment reagent) is in the range of 5 mM. After pre-dilution, and/or addition of an agent blocking DTT, and/or by way of dilution with a buffer comprising a first antibody, the concentration of DTT in oxidized form preferably is 1.5 mM or below. This way there is no effect of the reducing agent on any of the immunological reagents used.

As described above, ATP stabilizes the tetrameric form of hTK-1. The latter function of ATP makes it an attractive additive to, e.g., the pretreatment buffer. In one embodiment the pretreatment buffer comprises a reducing agent as discussed above and ATP. The concentration of ATP in the sample pretreatment step should not be below 1.25 mM. A good choice for the ATP concentration in the pretreatment step will be in the range from 2 mM to 20 mM.

As obvious from the Examples given, the value for the level of TK-1 is of great utility in the determination of a prognostic index for patients with DLBCL. In one embodiment the present disclosure relates to the use of a value for the level of TK-1 in the determination of a prognostic index for patients with DLBCL.

In one embodiment the present disclosure relates to a prognostic index (PI) for DLBCL comprising a value for the level of thymidine kinase 1 that is combined with the standard parameters (components) of any of the three PIs described in detail above.

In one embodiment the present invention relates to the use of a value for the level of TK-1, in combination with the other standard parameters of a prognostic index for patients with DLBCL, in determining said prognostic index. In one embodiment the present disclosure relates to the use of TK-1 in the determination of a prognostic index for patients with DLBCL wherein the value for the level of TK-1 is combined with the other parameters as used in IPI, age adjusted IPI, R-IPI, or NCCN-IPI. In one embodiment the present disclosure relates to the use of TK-1 in the determination of a prognostic index for patients with DLBCL wherein the value for the level of TK-1 is combined with the other parameters as used in R-IPI, or NCCN-IPI.

In one embodiment the value for the level of TK-1 is used with the proviso that that the value for the level of TK-1 is used to substitute the value for the level of LDH in such PI.

For any patient, especially for those recently diagnosed with DLBCL, TK-1 could be measured and compared to a pre-defined cutoff. If the measured value for the newly diagnosed patient is above the predefined cutoff, the patient would be considered to have a higher risk score and be more likely to experience an unfavorable disease outcome.

In one embodiment the present disclosure relates to a prognostic index (PI) for DLBCL comprising a value for the level of thymidine kinase 1.

In one embodiment the present disclosure relates to a prognostic index (PI) for DLBCL comprising a value for the level of thymidine kinase 1, wherein said PI is selected from IPI, age adjusted IPI, R-IPI, or NCCN-IPI. In one embodiment the present disclosure relates to a prognostic index (PI) for DLBCL comprising a value for the level of thymidine kinase 1, wherein said PI is selected from R-IPI, or NCCN-IPI.

These and other embodiments are disclosed and encompassed by the description and Examples of the present invention. Further literature concerning any one of the methods, uses and compounds to be employed in accordance with the present invention may be retrieved from public libraries and databases, using for example electronic devices. For example, the public database "Medline", available on the Internet, may be utilized, for example in the World Wide Web under ncbi.nlm.nih.gov/PubMed/medline.html. Further databases and addresses available in the World Wide Web, such as ncbi.nlm.nih.gov/, fmi.ch/biology/research_tools.html,tigr.org/, or infobiogen.fr/, are known to the person skilled in the art and can also be obtained using the address in the World Wide Web under lycos.com.

Unless otherwise defined, all technical and scientific terms used herein have the same meaning as commonly understood by one of ordinary skill in the art to which this invention belongs. In case of conflict, the patent specification, including definitions, will prevail.

All amino acid sequences provided herein are presented starting with the most N-terminal residue and ending with the most C-terminal residue (N→C), as customarily done in the art, and the one-letter or three-letter code abbreviations as used to identify amino acids throughout the present invention correspond to those commonly used for amino acids.

Certain aspects of the invention are also illustrated by way of the attached figures.

## Brief Description of the Figures

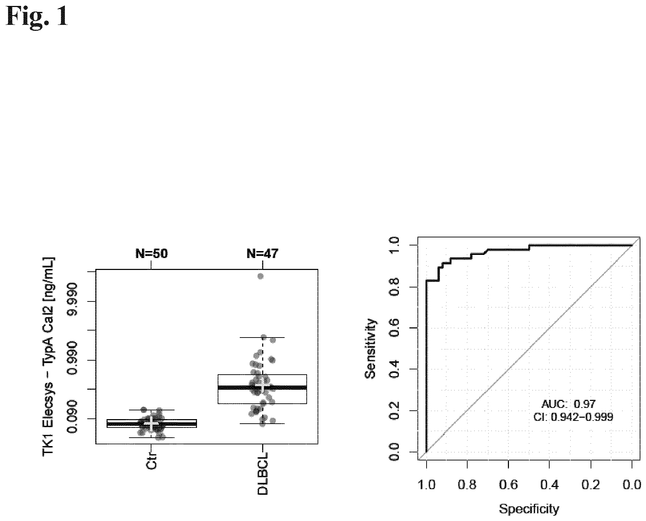

**Figure 1:** Graphical representation of the immunoassay data. In the left hand part of the Figure the Box-Whisker-Plots (Boxplots) are given. On the y-axis (in log scale) the concentration in ng/ml is shown. In the right hand part of the Figure the area under the curve (AUC) is shown. (Abbreviations: Ctr = control samples; DLBCL = samples from patients with diffuse large B-cell lymphoma).

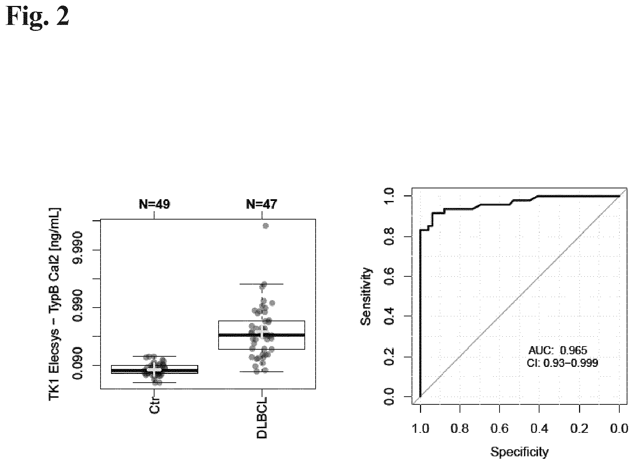

**Figure 2:** Graphical representation of the LIAISON® Thymidine Kinase (activity) assay data. In the left hand part of Fig: 2 Box-Whisker-Plots (Boxplots) with units per ml on the y-axis (in log scale) are given. In the right hand part of this Figure the area under the curve (AUC) is shown. (Abbreviations: Ctr = control samples; DLBCL = samples from patients with diffuse large B-cell lymphoma).

**Figure 3:** Method comparison The Deming Regression Fit is shown for the correlation between the LIAISON® Thymidine Kinase (activity) assay data x-axis and the prototype immunoassay y-axis of Figure 3.

The following Examples illustrate the invention:

## Example 1: Materials & general methods

*Protein chemistry and labeling techniques*

Standard protein chemistry and labeling techniques are provided e.g. in Hermanson, G. "Bioconjugate Techniques" 3rd Edition (2013) Academic Press.

*Bioinformatics*

Bioinformatics methods are provided in e.g. Keith J.M. (ed.) "Bioinformatics" Vol. I and Vol. II, Methods in Molecular Biology Vol. 1525 and Vol. 1526 (2017) Springer, and in Martin, A.C.R. & Allen, J. "Bioinformatics Tools for Analysis of Antibodies" in: Dübel S. & Reichert J.M. (eds.) "Handbook of Therapeutic Antibodies" Wiley-VCH (2014).

*Electrochemiluminescent immunoassays*

Immunoassays and related methods are provided in e.g. Wild D. (ed.) "The Immunoassay Handbook" 4th Edition (2013) Elsevier. Ruthenium complexes as electrochemiluminescent labels are provided in e.g. Staffilani M. et al. Inorg. Chem. 42 (2003) 7789-7798. Typically, for the performance of electrochemiluminescence (ECL) based immunoassays an Elecsys 2010 analyzer or a successor system was used, e.g. a Roche analyzer (Roche Diagnostics GmbH, Mannheim Germany) such as E170, cobas e 601 module, cobas e 602 module, cobas e 801 module, and cobas e 411, and Roche Elecsys assays designed for these analyzers, each used under standard conditions, if not indicated otherwise.

## Example 2: Anti-hTK-1 antibodies

**hTK-1, clone 6C6, heavy chain:** (SEQ ID NO:3)

METGLRWLLLVAVLKGVQCQEQLEESGGDLVKPEGSLTLTCTASRFSFSSSYW

ICWVRQAPGKGLEWIACIYAGDSGSSYYASWAKGRFTVSKTSSTTVTLQTTSL

TAADTATYFCARASVGAAYDYFALWGPGTLVTVSSGQPKAPSVFPLAPCCGD

TPSSTVTLGCLVKGYLPEPVTVTWNSG

**hTK-1, clone** 6C6, light chain: (SEQ ID NO:4)

MDTRAPTQLLGLLLLWLPGARCALVMTQTPASVEAAMGGTVTIKCQASEDV

SSHLAWYQQRPGQPPKLLIYGASDLASGVPSRFTGSGSGTQFTLAISDLECADA

ATYYCQGYYYISDSPYVFGGGTEVVVKGDPVAPTVLIFPPAADQVATGTVTI

VCVANKYFPDVTVTWEVDGTTQTTGIENSKTPQNSADCTYNLSSTLTLTSTQ

YNSHKEYTCKVTQGTTSVVQSFNRGDC

**hTK-1, clone** 4H4, heavy chain: (SEQ ID NO: 7)

METGLRWLLLVAVLKGVQCQSLEESGGGLVQPEGSLTLTCTASGFSFSSGYDM

CWVRQTPGKGLEWIACISVDSDGVTYYASWAKGRFTISKTSSTTVTLQMTSL

TAADTATYFCARGYESSSGVYIPYFTLWGPGTLVTVSSGQPKAPSVFPLAPCC

GDTPSSTVTLGCLVKGYLPEPVTVTWNSG

**hTK-1, clone** 4H4, light chain: (SEQ ID NO: 8)

MDMRAPTQLLGLLLLWLPGARCADIVLTQTPASVEAAVGGTVTIKCQASQSI

YSYLAWYQHKPGQPPKLLIYKASTLASGVPSRFKGSGSGTEYTLTISDLECADA

ATYYCQHYYYSSTSGGGVFGGGTEVVVKGDPVAPTVLIFPPAADQVATGTV

TIVCVANKYFPDVTVTWEVDGTTQTTGIENSKTPQNSADCTYNLSSTLTLTST

QYNSHKEYTCKVTQGTTSVVQSFNRGDC

**hTK-1, done 23C11, heavy chain:** (SEQ ID NO: 9)

METGLRWLLLVAVLKGVQCQSLEESGGRLVTPGTPLTLTCTASGFSLSNYYMS

WVRQAPGKGLEWIGIIYGDDNTYCANWTKGRFTISKTSTTVDLTITSPTTEDT

ATYFCARGPDYIAAKMDIWGPGTLVTVSLGQPKAPSVFPLAPCCGDTPSSTVT

LGCLVKGYLPEPVTVTWNSG

**hTK-1, clone 23C11, light chain:** (SEQ ID NO: 10)

MDTRAPTQLLGLLLLWLPGARCDVVMTQTPASVEAAVGGTVTIKCQASQSIS

GYLSWYQQKPGQRPKLLIYRASTLESGVPSRFKGSGSGTEFTLTISDLECADAA

TYYCQCTYGSSTFSSYGNAFGGGTEVVVKGDPVAPTVLIFPPAADQVATGTV

TIVCVANKYFPDVTVTWEVDGTTQTTGIENSKTPQNSADCTYNLSSTLTLTST

QYNSHKEYTCKVTQGTTSVVQSFNRGDC

As obvious full length immunoglobulins or any binding fragments thereof - if desired/required - can be easily construed by any person of skill in the art based on sequences disclosed above.

**Example 3: Epitope characterization**

As described in Example 2, four different monoclonal antibodies could be generated that exhibit the required binding properties needed for them to be of utility in an immunoassay development.

In a first attempt to characterize the epitope bound by the newly generated antibodies a PepScan analysis was performed. For this analysis synthetic peptides, consisting of 15 amino acids each, each shifted by 1 amino acid (1-15: 2-16, etc.) and spanning the entire sequence of hTK-1 (SEQ ID NO:1) were synthesized. These PepScan peptides were spotted onto microscope slides. After blocking for non-specific binding cell culture supernatant of the various MABs was incubated on the microscope slides. Unbound MAB was washed off and bound MAB was detected by use of HRP - labeled goat anti-rabbit IgG according to a routine method.

Only one of the four MABs (antibody 4H11) obtained by the method of Example 2 did react with a linear epitope. As could be shown the epitope bound by this antibody is comprised in the sequence spanning amino acid residues 211 through 230 of hTK-1 (SEQ ID NO: 6).

Polyclonal as well as monoclonal antibodies reacting with a synthetic peptide corresponding to a polypeptide consisting of amino acids 194 through 225 of hTK-1 (SEQ ID NO:2) are known in the prior art, see e.g. WO 2015/094106. The three MABs 4H4; 6C6; and 23C11, respectively, did not bind to a polypeptide consisting of amino acids 194 through 225 of hTK-1 (SEQ ID NO:2) nor did they show significant binding to any of the PepScan peptides tested. This indicates that these three MABs all bind to a conformation dependent epitope on hTK-1. As these three MABs from the very beginning looked quite promising for further assay development, additional efforts were made in order to gain more knowledge on the epitope bound by these three MABs.

The epitope characterization by competition experiments was performed on a GE Healthcare Biacore 4000 instrument at 25 °C. A Biacore Biotin Capture Kit, Series S sensor (Cat.-No.28-9202-34) was mounted into the instrument and was hydrodynamically addressed and preconditioned according to the manufacturer's instructions. The system buffer was HBS-N (10 mM HEPES pH 7.4, 150 mM NaCl). The sample buffer was the system buffer. The biotin capture reagent, as provided by the manufacturer GE Healthcare, was diluted 1:50 in system buffer and was injected at 10 μl/min for 60 sec over flow cells 1, 2, 3 and 4 to address the spots 1, 2 and 4, 5. Spot 3 served as a reference. 10 nM biotinylated primary antibody was injected at 30 μl/min for 120 sec contact time to address the spots 1 and 5 in all four flow cells. Spots 2 and 4 served as controls. 10 nM human recombinant thymidine kinase-1 (hTK-1, Roche, 114 kDa, tetramer) were injected at 30 μl/min into all flow cells for 180 sec contact time to address the spots 1, 2 and 4, 5. 100 nM of non-biotinylated primary antibody were again injected at 30 μl/min to address the spots 1, 2 and 4, 5 on all flow cells for 180 sec contact time in order to block remaining accessible epitopes of the primary antibody. 100 nM of secondary antibody was injected at 30 μl/min for 180 sec contact time into all flow cells to address the spots 1,2 and 4,5. Finally the complexes formed on the sensor surface were completely removed by a 120 sec contact time regeneration step over all flow cells and all spots using the regeneration solution as provided by the manufacturer GE Healthcare.

Four recombinant monoclonal rabbit IgG antibodies were this way investigated for their hTK-1 epitope accessibility properties: Antibody 4H11 (an antibody binding to an epitope comprised in amino acids 211 to 230 (SEQ ID NO:6) of hTK-1 and rabbit MABs 23C11, 6C6 and 4H4.

Before and after each sample injection, report points were set. The read out of the report points in Response Units [RU] was done by using the Biacore Evaluation V.1.1 software.

To the initial biotinylated primary antibody capturing signal (bi-Ab1, [RU]) the second binding response signal of the non-biotinylated primary antibody (block Ab1 [RU]) was added. The Molar Ratio Epitope Accessibility $MR_{EA}$ = Ab2 [RU] / (bi-Ab1 [RU] + block Ab1 [RU]) was calculated and was used as an estimate for the epitope accessibility of the respective antibodies used in the assay.

In order to verify the tetrameric state of the hTK analyte, a second Molar Ratio was calculated from the hTK binding signal versus the capture level of the biotinylated primary antibody by using the formula MR = hTK [RU] / bi-Ab1 [RU] * Molecular Weight bi-Ab1(150 kDa) / Molecular Weight hTK (114 kDa).

For example, antibody 4H11 showed a binding stoichiometry (=Molar Ratio) antibody 4H11 / hTK-1 MR of 1:1. In this biosensor assay a single, tetrameric hTK-1 molecule binds to a single antibody 4H11 molecule. Within the described antibodies, only antibody 4H11 shows a homologous hTK-1 complex formation when being used as block Ab. Therefore, a sandwich assay would be possible by using a sequential assay protocol using antibody 4H11 twice. No homologous complex formation could be detected for the rabbit monoclonal antibodies MABs 23C11, 6C6 and 4H4. This means that MABs 23C11, 6C6 and 4H4 bind to the same epitope region. Antibodies 23C11, 6C6 and 4H4 form a sandwich with antibody 4H11 as secondary antibody. According to this assay the best performing sandwich pair is 6C6 as biotinylated primary antibody, which forms a complex with antibody 4H11 showing a Molar Ratio $MR_{EA}$ = 0.4, which means 40 % epitope accessibility on the hTK analyte.

As obvious from the table shown below, the antibody 4H11 (binding to a C-terminal linear epitope comprised in SEQ ID NO:6) is able to form immuno complexes with 23C11, 6C6 and 4H4. On the other hand it is clear that the rabbit monoclonal antibodies MABs 23C11, 6C6 and 4H4 share the same epitope (see below Tab. 3).

*Table 3: Epitope Accessibility Matrix*

| | secondary antibodies | | | |
|---|---|---|---|---|
| biotinylated primary antibodies | 4H11-IgG | 23C11-IgG | 6C6-IgG | 4H4-IgG |
| 4H 11-IgG | 0.1 | 0.1 | 0.1 | 0.1 |
| 23C11-IgG | 0.2 | 0.0 | 0.0 | 0.0 |
| 6C6-IgG | 0.4 | 0.0 | 0.0 | 0.0 |
| 4H4-IgG | 0.2 | 0.0 | 0.0 | 0.0 |

Shown is the sandwich formation of four anti-hTK-1 antibodies using recombinant h-TK-1 as an analyte in solution. A value of 0.0 in the table indicates that the first and the second antibody used bind to the same epitope. A value of 0.1 or higher indicates sandwich formation, despite the intermediate blocking step, i.e. the two antibodies investigated bind to different epitopes.

**Example 4 : Production of the MAB-conjugates for use in Elecsys Immunoassay Experiments**

The procedures used are familiar to the skilled artisan. It therefore is considered redundant to give experimental details.

In brief, the following procedures/steps were carried out in order to obtain the antibody conjugates for the capture and detection sides of the immunological assay.

Cell culture supernatant (the recombinant antibodies comprised therein) as obtained from the by B-cell PCR generated cells (see above) was used as a starting material.

The recombinant antibody comprised in the tissue culture supernatant was purified by affinity chromatography to protein A.

An antibody used as a capture antibody was cleaved to the F(ab')2 fragment with pepsin and the F(ab')2 fragment further purified by affinity chromatography and size-exclusion chromatography. The F(ab')2 fragment was then reduced to Fab' and site-specific biotinylated via thiol-chemistry, thereby obtaining a mono-biotinylated Fab'-fragment.

An antibody used as a detection antibody was chemically conjugated to sulfo-Ruthenium (WO 2003/002974) by use of a sulfo-BPRu NHS Ester ( = CAS Reg. Number 482618-42-8 also known in the art as ruthenate(2-), bis[[2,2'-bipyridine]-4, 4'-dimethanesulfonato(2-)-$\kappa N^1,\kappa N^1$][1-[4-(4'-methyl[2,2'-bipyridin]-4-yl-$\kappa N^1,\kappa N^1$) -1-oxobutoxy]-2,5-pyrrolidinedione]-, sodium (1:2), (OC-6-31 )) and unbound label was removed by size-exclusion chromatography.

**Example 5: Samples and hTK-1 measurements**

5.1 Samples

A "black-and-white" panel was investigated. On the one hand serum samples from 50 (for some experiments only 49 were still available) healthy donors have been used for quantification of hTK-1 in various assays. On the other hand hTK-1 was measured in 48 (for some experiments only 47 were still available) samples from patients with diffuse large B-cell lymphoma (DLBCL).

## 5.2 Prototype electrochemiluminescence immunoassays

Based on the monoclonal rabbit antibodies obtained as described in Example 3, purified and conjugated as described in Example 4, respectively, several prototype immunoassays have been established.

The typical set-up of a prototype electrochemiluminescent immunoassay makes use of biotinylated capture antibody (or an antigen binding fragment thereof) and a detection antibody (or an antigen binding fragment thereof) which is labeled with a ruthenium complex.

Immunoassay data in most cases were generated using a conventional Fab' fragment that was biotinylated according to conventional procedures. Measurements were carried out in a sandwich assay format on a cobas® E170 analyzer from Roche. Signal detection in the cobas® E170 analyzer is based on electrochemiluminescence. In this sandwich assay the biotin-conjugate (i.e. the capture antibody) is immobilized on the surface of a streptavidin-coated magnetic bead. The detection-antibody bears a complexed ruthenium cation as the signaling moiety. In the presence of analyte, the chromogenic ruthenium complex is bridged to the solid phase and emits light at 620 nm after excitation at the platinum electrode comprised in the measuring cell of the cobas® E170 analyzer. The signal output is in arbitrary light units.

Measurements were, e.g., performed with calibrators spiked with recombinant hTK-1 from HEK-cells as well as with the human serum samples mentioned above.

The experimental hTK-1 assay was conducted as follows. 25 μl of human serum sample or of spiked calibrator, 25 μl of pretreatment reagent (comprising 10 mM DTBA) were mixed an incubated for 9 minutes; thereafter 60 μl of capture antibodybiotin conjugate and 60 μl of detection antibody ruthenium label conjugate were incubated together for another 9 minutes followed by the addition of 30 μl streptavidin-coated paramagnetic microparticles. The final mixture was incubated for further 9 minutes. Afterwards, the hTK-1 was detected as usual (i.e. via the electrochemiluminescent signal generated in these experiments).

Below given are the results obtained for the combination of biotinylated 6C6 (used as Fab'-Bi) with ruthenylated 4H11 (used as IgG).

Calibration results are given in Table 4 below.

*Table 4: Measurement of hTK-1 with the immunoassay prototype*

| [hTK-1] in ng/mL | TK-1: Prototype Assay<br><br>6C6-Fab'-Bi : 4H11-IgG-suBPRu | | |
| --- | --- | --- | --- |
| | Counts | Conc | MW$_{conc}$ |
| 0.0000 | 1837 | 0.0000 | **0.0000** |
| | 1826 | 0.0000 | |
| 1.00 | 30193 | 1.30 | **1.30** |
| | 30103 | 1.30 | |
| 5.00 | 105843 | 4.92 | **4.91** |
| | 105452 | 4.90 | |
| 10.0 | 198859 | 9.47 | **9.60** |
| | 204252 | 9.73 | |
| 100 | 1960303 | 99.5 | **100** |
| | 1994291 | 101 | |

In Table 4 signals obtained in the prototype immunoassay using various amounts of recombinant hTK-1 as analyte are given. Double measurements have been performed.

Box-Whisker-Plots have been calculated, the receiver-operator-characteristic (ROC) has been analyzed and the area under the curve (AUC) was determined. For the prototype immunoassay the AUC was 0.965. Both Box-Whisker-Plots (BoxPlots) with the determined concentrations of hTK-1 and the AUCs are shown in Figure 1.

5.3 DiaSorin TK-1 activity assay

The LIAISON® Thymidine Kinase assay, manufactured by DiaSorin, is an indirect, modified two-step, competitive chemiluminescence immunoassay (CLIA) for the quantitative determination of TK in human serum and EDTA plasma. The LIAISON® Thymidine Kinase assay was performed according to the instructions given by the manufacturer with 50 control samples and 48 samples from patients with DLBCL. It utilizes an initial enzymatic reaction in which TK in the sample converts AZT (3'-azido-3'-deoxythymidine) to AZTMP (3'-azido-3'-deoxythymidine mono phosphate), this is followed by a competitive immunoassay for the quantitative determination of AZTMP. The amount of AZT converted to AZTMP is a measure of the amount of TK present in the sample. In the assay, 50 µL of sample is incubated with 100 µL of Assay Buffer 1, 20 µL of Assay Buffer 2, and 20 µL of paramagnetic particles coated with anti-AZTMP polyclonal antibody. Rabbit anti-goat IgG, then anti-AZTMP goat polyclonal is coated to the solid phase. This is incubated for 40 minutes and then 100 µL of tracer, an AZTMP analogue conjugated to an isoluminol derivative is added. During the first incubation, AZTMP binds to the solid phase. In the second incubation, the tracer conjugate competes for binding with the AZTMP in the solution. After a 20 minute incubation, the unbound material is removed with a wash cycle. The starter reagents are then added and a flash chemiluminescent reaction is initiated. The light signal is measured by a photomultiplier as relative light units (RLU) and is proportional to the concentration of TK present in calibrators, controls, or samples.

Box-Whisker-Plots have been calculated, the receiver-operator-characteristic (ROC) has been analyzed and the area under the curve (AUC) was determined. For LIAISON® Thymidine Kinase assay the AUC was found to be 0.958. Both Box-Whisker-Plot (Boxplot) and the AUC are shown in Figure 2.

## Example 6: Comparison of TK-1 values as determined with activity assay / immunoassay

The values obtained with LIAISON® Thymidine Kinase assay on the one hand and the prototype immunoassay on the other hand were compared to each other. Taking into account that one assay measure thymidine kinase activity while the other measures the amount of immunoreactive hTK-1 a surprisingly high correlation (in the range of 0.95 or even above - dependent on the statistical method used) between the two different assays was found. The good correlation between these different assays for hTK-1 is also quite obvious from Figure 3.

## Example 7: Use of TK-1 values as key element of a prognostic index

### 7.1 Analytical approach

The prognostic ability of thymidine kinase 1 (TK-1) was retrospectively assessed with samples from the pharmacological MAIN study, which was conducted with patients suffering from diffuse large B-cell lymphoma (DLBCL) [Seymour et al., 2014]. TK-1 concentrations in serum were measured with the assay termed prototype B) in Example 5.2 for each of the 407 baseline samples that were still available. The progression-free survival (PFS) event rate within this subset was highly similar to the PFS of the total study population (both approximately 31%).

In order to have a fair comparison of all multivariate models, we created a data subset, such that only samples with all relevant data points available (TK-1 measurement and all index components) were included. This resulted in the final analysis data set containing 370 samples, of which 116 stemmed from patients who exhibited a PFS event within three years after R-CHOP treatment start. Due to the small number of samples with an event-free follow-up time longer than three years, samples were censored at the 3-year mark.

In order to assess whether the prognostic value of TK-1 is independent from known clinical and demographic risk factors, Cox proportional hazard models for each of the three described prognostic indices including the respective index's risk components plus log2-transformed TK-1 values were calculated.

### 7.2 Contribution of TK-1 to R-IPI and NCCN-IPI

To assess the ability of TK-1 to improve the existing risk scores for prognosis of PFS, the index components were either extended with log2-transformed TK-1 values, or the LDH component was replaced with the log2 TK-1 values. Both extension and substitution was performed by creating a Cox portioned hazard model including the respective risk components as independent variables, and comparing the c-indices of the original models (R-IPI and NCCN-IPI) with the c- indices of TK-1-extended models (with and without the LDH component, respectively). The significance in terms of p-values of the respective differences were assessed with a bootstrap resampling approach.

### a) TK-1 as covariate in the R-IPI

Results of the statistical analysis of the contribution provided by TK-1 to the R-IPI are given below in Table 5.

*Table 5: Multivariate Cox proportional hazard model including R-IPI's risk components binarized according to the thresholds in Table 1 plus TK-1 (log2-transformed). The hazard ratios are given along with their respective 95% confidence interval and the p-value*

| Covariate | Hazard ratio (HR) | 95%-CI HR | p-value |
|---|---|---|---|
| IPI.Age (bin) | 1.083 | 0.750-1.564 | 0.671 |
| IPI.AnnArborStage (bin) | 1.577 | 0.971-2.561 | 0.066 |
| IPI.ECOG (bin) | 1.829 | 1.226-2.731 | 0.003 |
| IPI.LDH (bin) | 0.912 | 0.582-1.429 | 0.688 |
| IPI.ExtranodalSites (bin) | 1.183 | 0.785-1.784 | 0.422 |

(continued)

| Covariate | Hazard ratio (HR) | 95%-CI HR | p-value |
|---|---|---|---|
| TK-1 (log2) | 1.307 | 1.127-1.516 | <0.001 |

The Cox proportional hazard model combining the binary R-IPI risk components with TK-1 (log2-transformed) clearly shows that TK-1 substantially adds prognostic information to the model. The hazard ratio (HR) is approximately 1.3 with the smallest p-value of all model components of <0.001 (see Table 5). The hazard ratio can be interpreted in a way that a 2-fold increase of TK-1 is associated with a 1.3-fold increase of risk for a PFS event (i.e. disease progression or death).

For the trichotomized TK-1 scenario, TK-1 concentrations were first stratified into three groups (i.e. low, intermediate and high levels). The optimal cutoffs were demined in a univariate setting by maximizing the logrank statistic (i.e. maximizing the different between the three survival curves in a Kaplan-Meier analysis), while ensuring that at least 10 samples were present in each group. The herewith determined ranges are:

- TK-1 low $\quad$ = $\quad$ 0% - 24% $\quad$ (0 - 0.7 ng/ml)

- TK-1 intermed. $\quad$ = $\quad$ 24.1% - 86% $\quad$ (0.71 - 3.5 ng/ml)

- TK-1 high $\quad$ = $\quad$ 86.1% - 100% $\quad$ (above 3.5 ng/ml)

Based on the method comparison between Roche Elecsys TK-1 concentration and Diasorin TK-1 activity measurements, these ranges can be further expressed in terms of U/L based on the relationship 1 ng/ml TK-1 (Roche) $\approx$ 25 U/L TK-1 (Diasorin). The mean activity in healthy individuals was reported to be 4.3 U/L by Diasorin, which is used as a relative reference for the cutoffs:

- TK-1 low $\quad$ = $\quad$ 0 – 4 times the mean $\quad$ (0 – 18 U/L)

- TK-1 intermed. $\quad$ = $\quad$ 4 – 20 times the mean $\quad$ (17.6 – 88 U/L)

- TK-1 high $\quad$ = above 20 times the mean $\quad$ (above 88 U/L)

In the multivariate Cox model, the trichotomized TK-1 values significantly contribute to the prognostic ability with a p-value of 0.013 and <0.001 for intermediate versus low and high versus low TK-1 levels, respectively (see Table 6). The corresponding hazard ratios are 2.11 and 5.40, meaning that a patient with an intermediate TK-1 level has an approximately 2.1-fold higher risk for a PFS event over low-TK-1 patients; and a patient with a high TK-1 level even 5.4-fold.

Table 6: Multivariate Cox proportional hazard model including R-IPI's risk components binarized according to the thresholds in Table 1 plus TK-1 (trichotomized). The hazard ratios are given along with their respective 95% confidence interval and the p-value.

| Covariate | Hazard ratio (HR) | 95%-CI HR | p-value |
|---|---|---|---|
| IPI.Age (bin) | 1.040 | 0.720-1.503 | 0.834 |
| IPI.AnnArborStage (bin) | 1.582 | 0.978-2.560 | 0.062 |
| IPI.ECOG (bin) | 1.939 | 1.302-2.889 | 0.001 |
| IPI.LDH (bin) | 0.813 | 0.522-1.268 | 0.362 |
| IPI.ExtranodalSites (bin) | 1.163 | 0.771-1.753 | 0.471 |
| TK-1.intermed (bin) | 2.113 | 1.171-3.814 | 0.013 |

(continued)

| Covariate | Hazard ratio (HR) | 95%-CI HR | p-value |
|---|---|---|---|
| TK-1.high (bin) | 5.399 | 2.659-10.96 | <0.001 |

b) TK-1 as a covariate in the NCCN-IPI

Results of the statistical analysis of the contribution provided by TK-1 to the NCCN-IPI are given below in Table 7.

Table 7: Multivariate Cox proportional hazard model including NCCN IPI's risk components binarized according to the thresholds in Table 2 plus TK-1 (log2-transformed). The hazard ratios are given along with their respective 95% confidence interval and the p-value

| Covariate | Hazard ratio (HR) | 95%-CI HR | p-value |
|---|---|---|---|
| NCCN.Age40-60 (bin) | 1.978 | 0.962-4.064 | 0.064 |
| NCCN.Age60-75 (bin) | 1.569 | 0.762-3.229 | 0.221 |
| NCCN.Age>75 (bin) | 3.377 | 1.437-7.932 | 0.005 |
| NCCN.LDH1-3 (bin) | 0.967 | 0.612-1.527 | 0.886 |
| NCCN.LDH>3 (bin) | 1.557 | 0.708-3.424 | 0.271 |
| NCCN.AnnArborStage (bin) | 1.593 | 0.986-2.575 | 0.057 |
| NCCN.ExtranodalDisease (bin) | 1.003 | 0.563-1.785 | 0.993 |
| NCCN.ECOG (bin) | 1.795 | 1.201-2.682 | 0.004 |
| TK-1 (log2) | 1.248 | 1.056-1.476 | 0.009 |

The multivariate model combining the binary NCCN-IPI risk components with TK-1 (log2-transformed) also shows significant added prognostic value of TK-1 (p-value = 0.009, see Table 7). The hazard ratio (HR) is approximately 1.25, which is only slightly smaller than in the R-IPI model.

Results of the statistical analysis of the contribution provided by trichotomized TK-1 to the NCCN-IPI are given below in Table 8.

Table 8: Multivariate Cox proportional hazard model including NCCN IPI's risk components binarized according to the thresholds in Table 2 plus TK-1 (trichotomized). The hazard ratios are given along with their respective 95% confidence interval and the p-value.

| Covariate | Hazard ratio (HR) | 95%-CI HR | p-value |
|---|---|---|---|
| NCCN.Age40-60 (bin) | 1.899 | 0.923-3.908 | 0.082 |
| NCCN.Age60-75 (bin) | 1.504 | 0.731-3.095 | 0.268 |
| NCCN.Age>75 (bin) | 2.975 | 1.261-7.016 | 0.013 |
| NCCN.LDH1-3 (bin) | 0.851 | 0.542-1.338 | 0.485 |
| NCCN.LDH>3 (bin) | 1.255 | 0.597-2.637 | 0.549 |
| NCCN.AnnArborStage (bin) | 1.614 | 1.001-2.602 | 0.050 |
| NCCN.ExtranodalDisease (bin) | 0.904 | 0.506-1.615 | 0.733 |
| NCCN.ECOG (bin) | 1.908 | 1.279-2.845 | 0.002 |
| TK-1.intermed (bin) | 2.148 | 1.193-3.868 | 0.011 |
| TK-1.high (bin) | 4.716 | 2.234-9.954 | <0.001 |

The model combining the binary NCCN-IPI risk components with TK-1 (trichotomized) shows significant added prognostic value of TK-1 (intermed versus low: p-value=0.011, high versus low: p-value<0.001; see Table 8). The corresponding HRs are 2.15 and 4.72, respectively, and are also very similar to the R-IPI counterpart.

7.3 TK-1 improves the prognostic ability of R-IPI as well as of NCCN-IPI

A widely used measure of a risk model's prognostic ability is the c-index, which can be regarded as the area under the ROC curve (AUC) analog in survival models. Table 9 shows the computed c-indices for the investigated prognostic indices (R-IPI and NCCN-IPI) along with the c-indices of the TK-1-extended models with and without excluding the respective binary LDH component. The inclusion of TK-1 improves these indices by 3.2% - 4%, regardless of the presence of LDH (see also Table 9). This improvement can be considered clinically relevant and is also statistically significant in most cases, although the study was not powered to show a significant improvement of the c-index (i.e. the bootstrapping-based confidence intervals do not include the 0, with the exception of NCCN-IPI vs NCCN-IPI + TK-1 (log2) w/o LDH and KPI vs KPI + TK-1(log2) w/o LDH (see Table 10)). This clearly indicates that TK-1 not only improves the investigated prognostic indices, but even has the potential to replace LDH in these indices.

*Table 9: c-indices of multivariate prognostic indices with TK-1 (+/- LDH)*

| Multivariate model | c-index |
|---|---|
| R-IPI | 0.626 |
| R-IPI + TK-1(log2) | 0.661 |
| R-IPI + TK-1(log2) w/o LDH | 0.660 |
| R-IPI + TK-1 (trichotomized) | 0.678 |
| R-IPI + TK-1 (trichotomized) w/o LDH | 0.674 |
| NCCN-IPI | 0.639 |
| NCCN-IPI + TK-1(log2) | 0.671 |
| NCCN-IPI + TK-1(log2) w/o LDH | 0.670 |
| NCCN-IPI + TK-1 (trichotomized) | 0.686 |
| NCCN-IPI + TK-1 (trichotomized) w/o LDH | 0.686 |

*Table 10: C-index differences between the reference models and the models including TK-1 (+/- LDH)*

| Index comparison | c-index diff | 95%-CI |
|---|---|---|
| R-IPI **vs** R-IPI + TK-1(log2) | 0.034 | 0.007 - 0.073 |
| R-IPI **vs** R-IPI + TK-1(log2) w/o LDH | 0.034 | 0.003 - 0.070 |
| R-IPI **vs** R-IPI + TK-1(trichotomized) | 0.052 | 0.020 - 0.096 |
| R-IPI **vs** R-IPI + TK-1(trichotomized) w/o LDH | 0.048 | 0.014 - 0.091 |
| NCCN-IPI **vs** NCCN-IPI + TK-1(log2) | 0.032 | 0.002 - 0.066 |
| NCCN-IPI **vs** NCCN-IPI + TK-1(log2) w/o LDH | 0.031 | -0.008 - 0.065 |
| NCCN-IPI **vs** R-IPI + TK-1(trichotomized) | 0.047 | 0.015 - 0.088 |
| NCCN-IPI **vs** R-IPI + TK-1(trichotomized) w/o LDH | 0.047 | 0.006 - 0.086 |

The data given in Tables 9 and 10, respectively, clearly show that TK-1 can be used in combination with existing prognostic indices to considerably improve the clinical scores in predicting disease outcome for DLBCL patients. The inclusion of TK-1 did improve each of the prognostic index investigated.

As also obvious from the Tables 9 and 10, TK-1 has even the potential to replace LDH within these indexes, since for two prognostic indices (R-IPI and KPI) the c-values are even slightly better if LDH is left out and for NCCN-IPI only slightly worse if LDH is not included.

For a new patient diagnosed to suffer from DLBCL, especially, before treatment is initiated, TK-1 should be measured and compared to a pre-defined cutoff. If the measured value for the new patient is above the predefined cutoff, the patient would be considered to have a higher risk score and be more likely to experience unfavorable disease outcome.

**Claims**

1.  A method of determining a prognostic index (PI) (also called risk score or PI-score) for a diffuse large B-Cell lymphoma (DLBCL) patient the method comprising

    a) determining at least the following parameters:

    i) extranodal disease status; ii) Ann Arbor stage; iii) the level of thymidine kinase 1; iv) age; and v) ECOG (Eastern Cooperative Oncology Group) performance status,
    wherein absence of extranodal disease values 0 points and presence of extranodal disease values 1 point,
    wherein an Ann Arbor stage of I or II values 0 points and an Ann Arbor stage of III or IV values 1 point,
    wherein a level of the thymidine kinase 1 up to and including the cut-off values 0 points and wherein a level of the thymidine kinase 1 above cut-off values at least 1 point,
    wherein if the age is below cut-off, the value is 0 points and if the age is above cut-off, the value is 1 point, and
    wherein, in case the ECOG performance status 1 or below, the value is 0 points and wherein, in case the ECOG performance status is 2 or above, the value is 1 point, and

    b) summing up the values for i), ii) and iii) in the determination of the PI.

2.  The method of claim 1, wherein the thymidine kinase 1 cut-off level is set to 4 times the mean value of the TK-1 level as determined in healthy individuals, wherein a thymidine kinase 1 level up to and including the cut-off values 0 points and wherein any TK-1 level above cut-off values 1 point.

3.  The method of claim 1, wherein the thymidine kinase 1 cut-off level is set to 4 times the mean value of the TK-1 level as determined in healthy individuals, wherein a low thymidine kinase 1 level, i.e. a level of TK-1 up to and including the cut-off values 0 points, an intermediate thymidine kinase 1 level, i.e. a level of TK-1 above the cut-off and up to and including the value corresponding to 20 times the mean value as determined in healthy individuals values 1 point, and a high thymidine kinase 1 level, i.e. a level of TK-1 of more than 20 times the mean value as determined in healthy individuals values 2 points.

4.  The method of claim 1, wherein the cut-off on the protein level for TK-1 is equivalent to and includes 18 U/l in TK-1 enzymatic activity.

5.  The method of claim 1, wherein the cut-off on the protein level is equivalent to and includes 18 U/l and wherein a level of TK-1 above 18 U/l and up to and including 88 U/l values 1 point and a level of TK-1 above 88 U/l values 2 points.

6.  The method according to claim 1, wherein if the age is below and including 60, the value is 0 points, wherein if the age is above 60, the value is 1 point in the determination of the PI.

7.  The method according to any of claims 1 to 5, wherein if the age is below and including 40, the value is 0 points, wherein if the age is above 40 and up to and including 60, the value is 1 point, wherein if the age is above 60 and up to and including 75, the value is 2 points and wherein if the age is above 75 the value is 3 points in the determination of the PI.

8.  The method according to any of claims 1 to 5, wherein if the age is below and including 60, the value is 0 points, wherein if the age is above 60, the value is 1 point in the determination of the PI, wherein, in case the ECOG performance status is 1 or below the value is 0 points, and wherein, in case the ECOG performance status is 2 or above, the value is 1 point in the determination of the PI.

9.  A computer-readable program product comprising instructions which, when the program is executed by a computer, causes the computer to carry out determining a prognostic index (PI) for DLBCL comprising values for the i) extranodal disease status; ii) Ann Arbor stage; iii) the level of thymidine kinase 1; iv) age; and v) ECOG (Eastern Cooperative Oncology Group) performance status;
    wherein the said PI is calculated as defined in any of claims 1 to 8.

10. The computer-readable program product of claim 9, wherein the program is implemented into an app.

11. The computer-readable program product of claim 9 or 10, wherein said PI is selected from IPI, age adjusted IPI, R-IPI, or NCCN-IPI.

**Patentansprüche**

1. Verfahren zum Bestimmen eines prognostischen Index (PI) (auch Risiko-Score oder PI-Score genannt) für einen Patienten mit diffus großzelligem B-Zell-Lymphom (DLBCL), wobei das Verfahren Folgendes umfasst

   a) Bestimmen mindestens der folgenden Parameter:

   i) extranodaler Erkrankungsstatus; ii) Ann-Arbor-Stadium; iii) Konzentration von Thymidinkinase 1; iv) Alter; und v) ECOG-(Eastern Cooperative Oncology Group)-Leistungsstatus,
   wobei die Abwesenheit einer extranodalen Erkrankung mit 0 Punkten bewertet wird und die Gegenwart einer extranodalen Erkrankung mit 1 Punkt bewertet wird,
   wobei ein Ann-Arbor-Stadium I oder II mit 0 Punkten bewertet wird und ein Ann-Arbor-Stadium III oder IV mit 1 Punkt bewertet wird,
   wobei eine Konzentration der Thymidinkinase 1 bis zu und einschließlich des Grenzwertes mit 0 Punkten bewertet wird und wobei eine Konzentration der Thymidinkinase 1 über dem Grenzwert mit mindestens 1 Punkt bewertet wird,
   wobei, wenn das Alter unter dem Grenzwert liegt, die Bewertung 0 Punkte ist, und wenn das Alter über dem Grenzwert liegt, die Bewertung 1 Punkt ist, und
   wobei im Falle des ECOG-Leistungsstatus 1 oder darunter, die Bewertung 0 Punkte ist und wobei im Falle des ECOG-Leistungsstatus 2 oder darüber, die Bewertung 1 Punkt ist, und

   b) Summieren der Bewertungen für i), ii) und iii) bei der Bestimmung des PI.

2. Verfahren nach Anspruch 1, wobei die Thymidinkinase-1-Grenzwertkonzentration auf das 4-Fache des bei gesunden Individuen bestimmten durchschnittlichen Wertes der TK-1-Konzentration festgelegt wird, wobei eine Thymidinkinase-1-Konzentration von bis zu und einschließlich des Grenzwertes mit 0 Punkten bewertet wird und wobei eine TK-1-Konzentration über dem Grenzwert mit 1 Punkt bewertet wird.

3. Verfahren nach Anspruch 1, wobei die Thymidinkinase-1-Grenzwertkonzentration auf das 4-Fache des bei gesunden Individuen bestimmten durchschnittlichen Wertes der TK-1-Konzentration festgelegt wird, wobei eine niedrige Thymidinkinase-1-Konzentration, d. h. eine Konzentration von TK-1 bis zu und einschließlich des Grenzwertes, mit 0 Punkten bewertet wird, eine mittlere Thymidinkinase-1-Konzentration, d. h. eine Konzentration von TK-1 über dem Grenzwert und bis zu und einschließlich des Wertes, der dem 20-Fachen des bei gesunden Individuen bestimmten durchschnittlichen Wertes entspricht, mit 1 Punkt bewertet wird, und eine hohe Thymidinkinase-1-Konzentration, d. h. eine Konzentration von TK-1 von mehr als dem 20-Fachen des bei gesunden Individuen bestimmten durchschnittlichen Wertes, mit 2 Punkten bewertet wird.

4. Verfahren nach Anspruch 1, wobei der Grenzwert für TK-1 auf Proteinebene 18 E/l in TK-1-Enzymaktivität gleicht und einschließt.

5. Verfahren nach Anspruch 1, wobei der Grenzwert auf Proteinebene 18 E/l gleicht und einschließt und wobei eine Konzentration von TK-1 über 18 E/l und bis zu und einschließlich 88 E/l mit 1 Punkt bewertet wird und eine Konzentration von TK-1 über 88 E/l mit 2 Punkten bewertet wird.

6. Verfahren nach Anspruch 1, wobei, wenn das Alter weniger als und einschließlich 60 beträgt, die Bewertung 0 Punkte ist, wobei, wenn das Alter über 60 liegt, die Bewertung bei der Bestimmung des PI 1 Punkt ist.

7. Verfahren nach einem der Ansprüche 1 bis 5, wobei, wenn das Alter weniger als und einschließlich 40 beträgt, die Bewertung 0 Punkte ist, wobei, wenn das Alter über 40 liegt und bis zu und einschließlich 60 beträgt, die Bewertung 1 Punkt ist, wobei, wenn das Alter über 60 liegt und bis zu und einschließlich 75 beträgt, die Bewertung 2 Punkte ist, und wobei, wenn das Alter über 75 liegt, die Bewertung bei der Bestimmung des PI 3 Punkte ist.

8. Verfahren nach einem der Ansprüche 1 bis 5, wobei, wenn das Alter weniger als und einschließlich 60 beträgt, die Bewertung 0 Punkte ist, wobei, wenn das Alter über 60 liegt, die Bewertung bei der Bestimmung des PI 1 Punkt ist, wobei für den Fall, dass der ECOG-Leistungsstatus 1 oder weniger beträgt, die Bewertung 0 Punkte ist, und wobei für den Fall, dass der ECOG-Leistungsstatus 2 oder mehr beträgt, die Bewertung bei der Bestimmung des PI 1 Punkt ist.

9. Computerlesbares Programmprodukt, umfassend Anweisungen, die bei der Ausführung des Programms von einem Computer den Computer veranlassen, das Bestimmen eines prognostischen Index (PI) für DLBCL auszuführen, welcher Bewertungen für i) den extranodalen Erkrankungsstatus; ii) das Ann-Arbor-Stadium; iii) die Konzentration von Thymidinkinase 1; iv) das Alter; und v) den ECOG-(Eastern Cooperative Oncology Group)-Leistungsstatus umfasst;

wobei der PI wie in einem der Ansprüche 1 bis 8 definiert berechnet wird.

10. Computerlesbares Programmprodukt nach Anspruch 9, wobei das Programm in einer App implementiert ist.

11. Computerlesbares Programmprodukt nach Anspruch 9 oder 10, wobei der PI aus IPI, altersbereinigtem IPI, R-IPI oder NCCN-IPI ausgewählt ist.


## Revendications

1. Procédé de détermination d'un indice de pronostic (IP) (également appelé score de risque ou score IP) pour un patient atteint d'un lymphome diffus à grandes cellules B (DLBCL), le procédé comprenant

   a) la détermination d'au moins les paramètres suivants :

   i) le statut de maladie extranodale ; ii) le stade d'Ann Arbor ; iii) le taux de thymidine kinase 1 ; iv) l'âge ; et v) le statut de performance ECOG (Eastern Cooperative Oncology Group),
   dans lequel l'absence de maladie extranodale vaut 0 point et la présence de maladie extranodale vaut 1 point, dans lequel un stade d'Ann Arbor de I ou II vaut 0 point et un stade d'Ann Arbor de III ou IV vaut 1 point, dans lequel un taux de thymidine kinase 1 allant jusqu'au et incluant le seuil vaut 0 point et dans lequel un taux de thymidine kinase 1 supérieur au seuil vaut au moins 1 point, dans lequel, si l'âge est inférieur au seuil, la valeur est de 0 point et si l'âge est supérieur au seuil, la valeur est de 1 point, et
   dans lequel, dans le cas du statut de performance ECOG inférieur ou égal à 1, la valeur est de 0 point et dans lequel, dans le cas du statut de performance ECOG supérieur ou égal à 2, la valeur est de 1 point, et

   b) le calcul de la somme des valeurs pour i), ii) et iii) dans la détermination de l'IP.

2. Procédé selon la revendication 1, dans lequel le taux seuil de thymidine kinase 1 est fixé à 4 fois la valeur moyenne du taux de TK-1 telle que déterminée chez les individus sains, dans lequel un taux de thymidine kinase 1 allant jusqu'au et incluant le seuil vaut 0 point et dans lequel un taux quelconque de TK-1 supérieur au seuil vaut 1 point.

3. Procédé selon la revendication 1, dans lequel le taux seuil de thymidine kinase 1 est fixé à 4 fois la valeur moyenne du taux de TK-1 telle que déterminée chez les individus sains, dans lequel un taux de thymidine kinase 1 faible, c'est-à-dire un taux de TK-1 allant jusqu'au et incluant le seuil vaut 0 point, un taux de thymidine kinase 1 intermédiaire, c'est-à-dire un taux de TK-1 supérieur au seuil et allant jusqu'à et incluant la valeur correspondant à 20 fois la valeur moyenne telle que déterminée chez les individus sains vaut 1 point, et un taux de thymidine kinase 1 élevé, c'est-à-dire un taux de TK-1 supérieur à 20 fois la valeur moyenne telle que déterminée chez les individus sains vaut 2 points.

4. Procédé selon la revendication 1, dans lequel le seuil du taux de protéine pour TK-1 est équivalent à et inclut 18 U/l dans l'activité enzymatique de TK-1.

5. Procédé selon la revendication 1, dans lequel le seuil du taux de protéine est équivalent à et inclut 18 U/l et dans lequel un taux de TK-1 supérieur à 18 U/l et allant jusqu'à et incluant 88 U/l vaut 1 point et un taux de TK-1 supérieur à 88 U/l vaut 2 points.

6. Procédé selon la revendication 1, dans lequel si l'âge est inférieur à et incluant 60, la valeur est de 0 point, dans lequel si l'âge est supérieur à 60, la valeur est de 1 point dans la détermination de l'IP.

7. Procédé selon l'une quelconque des revendications 1 à 5, dans lequel si l'âge est inférieur à et incluant 40, la valeur est de 0 point, dans lequel si l'âge est supérieur à 40 et allant jusqu'à et incluant 60, la valeur est de 1 point, dans lequel si l'âge est supérieur à 60 et allant jusqu'à et incluant 75, la valeur est de 2 points et dans lequel si l'âge est supérieur à 75, la valeur est de 3 points dans la détermination de l'IP.

8. Procédé selon l'une quelconque des revendications 1 à 5, dans lequel si l'âge est inférieur à et incluant 60, la valeur est de 0 point, dans lequel si l'âge est supérieur à 60, la valeur est de 1 point dans la détermination de l'IP, dans lequel, dans le cas du statut de performance ECOG inférieur ou égal à 1, la valeur est de 0 point, et dans lequel, dans le cas du statut de performance ECOG supérieur ou égal à 2, la valeur est de 1 point dans la détermination de l'IP.

9. Produit de programme lisible par ordinateur comprenant des instructions qui, lorsque le programme est exécuté par un ordinateur, amènent l'ordinateur à réaliser la détermination d'un indice de pronostic (IP) pour le DLBCL comprenant les valeurs pour i) le statut de maladie extranodale ; ii) le stade d'Ann Arbor ; iii) le taux de thymidine kinase 1 ; iv) l'âge ; et v) le statut de performance ECOG (Eastern Cooperative Oncology Group) ;
dans lequel ledit IP est calculé tel que défini dans l'une quelconque des revendications 1 à 8.

10. Produit de programme lisible par ordinateur selon la revendication 9, dans lequel le programme est implémenté dans une application.

11. Produit de programme lisible par ordinateur selon la revendication 9 ou 10, dans lequel ledit IP est choisi parmi l'IPI, l'IPI ajusté à l'âge, le R-IPI ou le NCCN-IPI.

# Fig. 1

Fig. 2

**Fig. 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5221605 A
- US 5591581 A
- US 5597910 A
- WO 9005296 A
- WO 9214139 A
- WO 9005301 A
- WO 9624690 A
- US 9503190 W
- US 9716942 W
- US 9606763 W
- WO 9508644 A
- WO 9606946 A
- WO 9633411 A
- WO 8706706 A
- WO 9639534 A
- WO 9641175 A
- WO 9640978 A

- US 9703653 W
- US 437348
- US 5679519 A
- WO 2012107419 A
- US 20100111856 A
- US 5342606 A
- US 5428155 A
- US 5316757 A
- US 5480990 A
- US 5462725 A
- US 5428139 A
- US 5385893 A
- US 5739294 A
- US 5750660 A
- US 5834456 A
- WO 2015094106 A
- WO 2003002974 A

### Non-patent literature cited in the description

- **WIGHT et al.** *Blood Reviews,* 2018
- **ZIEPERT M. et al.** *Journal of Clinical Oncology,* 2010, vol. 28 (14), 2373
- **ZHOU et al.** *Blood,* 2014, vol. 123 (6), 837
- **KAZUHITO et al.** *Leukemia and Lymphoma,* 2013, vol. 54 (11), 2412-2417
- **SHIPP MA ; HARRINGTON DP.** A predictive model for aggressive non-Hodgkin's lymphoma. *The international non-Hodgkin's Lymphoma prognostic factors project; N. Engl. J. Med.,* 1993, vol. 329 (14), 987-94
- **OKEN MM ; CREECH RH ; TORMEY DC.** Eastern Cooperative Oncology Group, Robert Comis M.D., Group Chair. Toxicity and response criteria of the Eastern Cooperative Oncology Group. *Am J Clin Oncol.,* December 1982, vol. 5 (6), 649-55
- **BRIGGS et al.** Synthesis of Functionalised Fluorescent Dyes and Their Coupling to Amines and Amino Acids. *J. Chem. Soc., Perkin-Trans.,* 1997, vol. 1, 1051-1058
- **BRIGGS et al.** Synthesis of Functionalized Fluorescent Dyes and Their Coupling to Amines and Amino Acids. *J. Chem. Soc., Perkin-Trans.,* 1997, vol. 1, 1051-1058
- **DODEIGNE C. et al.** *Talanta,* 2000, vol. 51, 415-439
- **KNIGHT et al.** *Analyst,* 1994, vol. 119, 879-890

- **HNATOWICH et al.** *J. Immunol. Methods,* 1983, vol. 65, 147-157
- **MEARES et al.** *Anal. Biochem.,* 1984, vol. 142, 68-78
- **MIRZADEH et al.** *Bioconjugate Chem.,* 1990, vol. 1, 59-65
- **MEARES et al.** *J. Cancer,* 1990, (10), 21-26
- **IZARD et al.** *Bioconjugate Chem.,* 1992, vol. 3, 346-350
- **NIKULA et al.** *Nucl. Med. Biol.,* 1995, vol. 22, 387-90
- **CAMERA et al.** *Nucl. Med. Biol.,* 1993, vol. 20, 955-62
- **KUKIS et al.** *J. Nucl. Med.,* 1998, vol. 39, 2105-2110
- **VEREL et al.** *J. Nucl. Med.,* 2003, vol. 44, 1663-1670
- **CAMERA et al.** *J. Nucl. Med.,* 1994, vol. 21, 640-646
- **RUEGG et al.** *Cancer Res.,* 1990, vol. 50, 4221-4226
- **LEE et al.** *Cancer Res.,* 2001, vol. 61, 4474-4482
- **MITCHELL et al.** *J. Nucl. Med.,* 2003, vol. 44, 1105-1112
- **KOBAYASHI et al.** *Bioconjugate Chem.,* 1999, vol. 10, 103-111

- **MIEDERER et al.** *J. Nucl. Med.,* 2004, vol. 45, 129-137
- **DENARDO et al.** *Clinical Cancer Research,* 1998, vol. 4, 2483-90
- **; BLEND et al.** *Cancer Biotherapy & Radiopharmaceuticals,* 2003, vol. 18, 355-363
- **NIKULA et al.** *J. Nucl. Med.,* 1999, vol. 40, 166-76
- **KOBAYASHI et al.** *J. Nucl. Med.,* 1998, vol. 39, 829-36
- **MARDIROSSIAN et al.** *Nucl. Med. Biol.,* 1993, vol. 20, 65-74
- **ROSELLI et al.** *Cancer Biotherapy & Radiopharmaceuticals,* 1999, vol. 14, 209-20
- **HARLOW ; LANE.** Antibodies: A Laboratory Manual. Cold Spring Harbor Laboratory Press, 1988
- **HARLOW ; LANE.** Using Antibodies: A Laboratory Manual. Cold Spring Harbor Laboratory Press, 1999
- Pliickthun in The Pharmacology of Monoclonal Antibodies. Springer-Verlag, 1994, vol. 113, 269-315
- **HARLOW et al.** Antibodies: A Laboratory Manual. Cold Spring Harbor Laboratory, 1988
- **SHARIF et al.** *BMC Biochemistry,* 2012, vol. 13, 12
- **HERMANSON, G.** Bioconjugate Techniques. Academic Press, 2013
- Bioinformatics. vol. I,II
- Methods in Molecular Biology. Springer, 2017, vol. 1525,1526
- Bioinformatics Tools for Analysis of Antibodies. **MARTIN, A.C.R. ; ALLEN, J.** Handbook of Therapeutic Antibodies. Wiley-VCH, 2014
- The Immunoassay Handbook. Elsevier, 2013
- **STAFFILANI M. et al.** *Inorg. Chem.,* 2003, vol. 42, 7789-7798
- *CHEMICAL ABSTRACTS,* 482618-42-8